# EUROPEAN PATENT SPECIFICATION

(11) **EP 1 263 495 B1**
(45) Date of publication and mention of the grant of the patent: **30.08.2006**
(21) Application number: 01905066.5
(22) Date of filing: 26.01.2001
(51) Int. Cl.: A61M 37/00, A61M 1/10

(54) **CANNULATION SYSTEM**
KANNULATIONSANORDNUNG
SYSTEME DE CANNULATION

(30) Priority: 27.01.2000 US 178479 P
(43) Date of publication of application: 11.12.2002
(73) Proprietor: A-Med Systems, Inc., West Sacramento, CA 95691-3817 (US)
(72) Inventor: ABOUL-HOSN, Walid, N., Fair Oaks, CA 95628 (US); KANZ, William, Sacramento, CA 95822 (US); BAKER, Bruce, Placerville, CA 95667 (US); GUIDERA, Michael, Carmichael, CA 95628 (US); O'CONNELL, Desmond, Seattle, WA 98155 (US); KOSALEK, Kim, L., Sacramento, CA 95833 (US); NOOR, Sedig, Temecula, CA 92592 (US)
(74) Representative: Dee, Ian Mark
(86) International application number: PCT/US2001/002531
(87) International publication number: WO 2001/054749

(56) References cited:
- WO-A-94/13955
- WO-A-99/59652
- WO-A-99/65546
- US-A- 5 021 048
- US-A- 5 840 070
- US-A- 5 965 089
- US-A- 6 123 725

## Description

### BACKGROUND OF THE INVENTION

### I. Field of the Invention

The present invention relates generally to a cannulation system for transporting bodily fluids. More particularly, the present invention is directed to a cannulation system for augmenting the cardiac output of the heart during cardiac surgery.

### II. Discussion of the Prior Art

Major heart surgery is oftentimes accomplished by procedures that require full cardiopulmonary bypass (CPB) through the use of artificial heart-lung machines and complete cessation of cardiopulmonary activity. While the average mortality rate with this type of procedure is low, it is nonetheless associated with a complication rate that is often much higher compared to when cessation of the heart and CPB are not required. The use of CPB continues to represent a major assault on a host of body systems. For example, there is noticeable degradation of mental faculties following such surgeries in a significant percentage of patients who undergo coronary artery bypass grafting (CABG) procedures. The CABG procedure generally involves open chest surgical techniques to treat diseased vessels. During this procedure, the sternum of the patient is cut in order to spread the chest apart and provide access to the heart. During surgery the heart is stopped, and by the use of CPB blood is diverted from the lungs to an artificial oxygenator. In general CABG procedures, a source of arterial blood is then connected to a coronary artery downstream from the occlusion. The source of blood is often an internal mammary artery, and the target coronary artery is typically among the anterior or posterior arteries which may be narrowed or occluded. The degradation of mental faculties resulting from CABG procedures is commonly attributed to cerebral arterial blockage and emboli from debris in the blood generated by the use of CPB. At the same time, the dramatic increase in the life expectancy of the general population has resulted in patients that are more likely to be older and in poor health, with less cardiovascular, systemic, and neurologic reserve needed to recover from the trauma caused by the use of CPB. As a consequence, inflammatory, hemostatic, endocrinologic, and neurologic stresses are tolerated to a much lesser degree by a significant number of patients today, and play a more significant role in CPB-induced morbidity.

The combined statistics of postoperative morbidity and mortality continue to illustrate the shortcomings of CPB. The extracorporeal shunting and artificially induced oxygenation of blood activates a system wide roster of plasma proteins and blood components in the body including those that were designed to act locally in response to infection or injury. When these potent actors are disseminated throughout the body without normal regulatory controls, the entire body becomes a virtual battleground. The adverse hemostatic consequences of CPB also include prolonged and potentially excessive bleeding. CPB-induced platelet activation, adhesion, and aggregation also contribute to a depletion in platelet number, and is further compounded by the reversibly depressed functioning of platelets remaining in circulation. The coagulation and fibrinolytic systems both contribute to hemostatic disturbances during and following CPB. However, the leading cause of morbidity and disability following cardiac surgery is cerebral complications. Gaseous and solid micro and macro emboli, and less often perioperative cerebral hypoperfusion, produce neurologic effects ranging from subtle neuropsychologic deficits to fatal stroke. Advances in computed tomography, magnetic resonance imaging, ultrasound, and other imaging and diagnostic techniques have added to the understanding of these complications. But with the possible exception of perioperative electroencephalography, these technologies do not yet permit real time surgical adjustments that are capable of preventing emboli or strokes in the making. Doppler and ultrasound evaluation of the carotid artery and ascending aorta, and other diagnostic measures, can help identify surgical patients at elevated risk for stroke and are among the growing list of pharmacologic and procedural measures for reducing that risk.

CPB also affects various endocrine systems, including the thyroid gland, adrenal medulla and cortex, pituitary gland, pancreas, and parathyroid gland. These systems are markedly affected not only by inflammatory processes, but also by physical and biochemical stresses imposed by extracorporeal perfusion. Most notably, CPB is now clearly understood to induce euthyroid-sick syndrome which is marked by profoundly depressed triiodothyronine levels persisting for days following cardiothoracic surgery. The efficacy of hormone replacement regimens to counteract this effect are currently undergoing clinical investigation. By contrast, levels of the stress hormones epinephrine, norepinephrine, and cortisol are markedly elevated during and following CPB, and hyperglycemia is also possible.

Beating heart bypass surgery has been recognized as desirable because it has the possibility of avoiding the necessity of placing the patient on a full CPB system. However, attempts at beating heart bypass surgery have met with limited success and have essentially been limited to surgery on the anterior heart vessels due to problems which develop when the beating heart is lifted or displaced from its normal position in order to perform the beating heart surgery. Typically when the beating heart is lifted or manipulated in order to provide surgical access to posterior heart vessels, a number of difficulties are encountered. When the beating heart is lifted and manipulated, the right side of the heart tends to collapse, particularly the right auricle or atrium and frequently the right ventricle and/or pulmonary artery. When the right side of the heart collapses, pulmonary blood flow either ceases or becomes inadequate, thus forcing the use of CPB. Another difficulty encountered is that, even if the right side of the heart does not collapse, the pulmonary artery and/or the pulmonary vein frequently become crimped or kinked thus also impeding the pulmonary blood flow. Similarly, during the lifting and manipulation of the beating heart for lateral or posterior access, the left side of the heart, particularly the left auricle or left atrium can also collapse or partially collapse, thus impeding aortic circulatory blood flow. Further, when the beating heart is lifted or manipulated for beating heart surgery access or during catheterization or cannulation procedures, the heart may lapse into arrhythmia or disrhythmia or may arrest at least a portion of the time or most of the time that the surgery is being performed thus likewise impeding pulmonary blood flow and arterial circulatory blood flow. As a result, patients undergoing beating heart surgery are at risk of having to be placed on CPB on an emergency basis in the event that the pulmonary and/or circulatory blood flow is compromised during the surgery, which presents the CPB-induced side effects previously described.

WO 99/65546 describes a double cannula system which can be inserted into a body cavity to allow simultaneous inflow and outflow of body fluids through the lumen of an trower cannula and the annular space between the inner cannula and an outer cannula.

The medical community is currently performing more beating heart bypass surgery in an effort to avoid the use of full CPB. The need is increasing for apparatus systems, methods and associated equipment to enhance the capability and versatility of beating heart surgery and to avoid CPB procedures in any heart surgery. The present invention is directed at addressing this need.

### SUMMARY OF THE INVENTION

The present invention provides a cannulation according to claim 1 and involves a cannulation system suitable, by way of example, for use in providing right heart support during beating heart surgery. The cannulation system of the present invention comprises a coaxial cannula assembly coupled to a centrifugal blood pumping system. The centrifugal blood pumping system includes a miniature centrifugal blood pump, a motor, a magnetic drive cable assembly coupling the centrifugal blood pump to the motor, and a microcomputer-based control console communicatively coupled to the motor for controlling the operation of the motor and hence the centrifugal blood pump. The coaxial cannula assembly includes an inner cannula disposed generally coaxially through an outer cannula. The outer cannula is semi-rigid and equipped with a basket portion (having a plurality of fluid inlet apertures formed therein) and a bent distal portion or J-tip that extends distally from the basket portion. The inner cannula is generally flexible such that, during insertion into the heart, it will be guided by and extend past the distal opening of the outer cannula. The generally coaxial relation between the inner and outer cannulas defines a first blood flow path in the annular space between the exterior surface of the inner cannula and the interior surface of the outer cannula, and a second blood flow path within the lumen of the inner cannula. The inner and outer cannulas are preferably moveably displaceable relative to one another such that fluid inlet apertures formed in the basket portion of the outer cannula and the open fluid outlet in the distal end of the inner cannula may be selectively positioned at different locations within the heart. The centrifugal blood pump includes an inflow port coupled to the first blood flow path (i.e. outer cannula), and an outflow port coupled to the second blood flow path (i.e. inner cannula). The coaxial cannula assembly may be introduced into the heart such that fluid inlet apertures in the outer cannula are disposed in the right atrium, and the open distal end of the inner cannula is disposed in the pulmonary artery. Under the direction of the control console, the miniature centrifugal blood pump may be selectively operated to withdraw blood from the right atrium and to reroute this blood for delivery into the pulmonary artery. Providing right heart support in this fashion advantageously eliminates the need for cardiopulmonary bypass (CPB) during beating heart surgery.

### BRIEF DESCRIPTION OF THE DRAWINGS

FIG. 1 is a perspective view of a cannulation system according to one embodiment of the present invention, including a coaxial cannula assembly coupled to a centrifugal blood pumping system;
FIG. 2 is a partial sectional view illustrating the coaxial cannula assembly of the present invention disposed in an exemplary fashion within the heart for providing right heart support during beating heart surgery;
FIG. 3 is a side view of the coaxial cannula assembly of the present invention during assembly, including an outer cannula having a J-tip distal portion, an inner cannula, and a stylet to aid in placing the coaxial cannula assembly into the heart;
FIG. 4 is a side view of the coaxial cannula assembly of the present invention after assembly, wherein the inner cannula is slideably disposed through the outer cannula so as to extend past a distal opening in the J-tip portion thereof;
FIG. 5 is a side view illustrating the step of dipping the hydrogel-coated J-tip portion of the outer cannula in a bath of sterile physiologic fluid for facilitating the passage of the inner cannula through the now-lubricious interior of the J-tip portion;
FIG. 6 is a partial sectional perspective view of the miniature centrifugal pump of the present invention, detailing the female quick-connect fluid outflow port, the priming port, and the magnetic drive cable assembly coupled to a rotor;
FIG. 7 is a side view of the centrifugal pump assembly of the present invention, including miniature centrifugal blood pump, the inflow tubing, and magnetic drive cable assembly;
FIG. 8 is a cross-sectional view of the centrifugal pump assembly of the present invention taken along lines 8 - 8 in FIG. 7;
FIG. 9 is an upper perspective view of a first pump housing member forming part of the pump housing of the miniature centrifugal pump of the present invention;
FIG. 10 is a lower perspective view of the first pump housing member of the miniature centrifugal pump of the present invention;
FIG. 11 is an upper perspective view of a second pump housing member forming part of the pump housing of the miniature centrifugal pump of the present invention;
FIG. 12 is a lower perspective drawing of the second pump housing member of the miniature centrifugal pump of the present invention;
FIG. 13 is a top view of the second pump housing member of the miniature centrifugal pump of the present invention shown in FIGS. 11 and 12;
FIG. 14 is a cross-sectional view of the second pump housing member of the present invention taken along lines 14 - 14 in FIG. 13;
FIG. 15 is a cross-sectional view of the second pump housing member of the present invention taken along lines 15 - 15 in FIG. 13;
FIG. 16 is a side view of the magnetic drive cable assembly according to an exemplary embodiment of the present invention;
FIG. 17 is a cross-sectional view of the magnetic drive cable assembly of the present invention taken along lines 17 - 17 in FIG. 16;
FIG. 18 is an exploded perspective view of an impeller assembly for use within the miniature centrifugal pump according to an exemplary embodiment of the present invention;
FIG. 19 is a side view of the impeller assembly of the present invention as shown in FIG. 18;
FIG. 20 is a top view of the impeller assembly of the present invention as shown in FIGS. 18 and 19;
FIG. 21 is a cross-sectional view of the impeller assembly of the present invention taken along lines 21 - 21 in FIG. 20;
FIG. 22 is a graph setting forth, by way of example only, the flow versus pressure characterization of the centrifugal pump of the present invention dimensioned for use as a blood pump according to an exemplary of the present invention;
FIG. 23 is a view of the front of the control console according to one embodiment of the present invention; and
FIG. 24 is a view of the back of the control console according to one embodiment of the present invention.

### DESCRIPTION OF THE PREFERRED EMBODIMENT

Illustrative embodiments of the invention are described below. In the interest of clarity, not all features of an actual implementation are described in this specification. It will of course be appreciated that in the development of any such actual embodiment, numerous implementation-specific decisions must be made to achieve the developers' specific goals, such as compliance with system-related and business-related constraints, which will vary from one implementation to another. Moreover, it will be appreciated that such a development effort might be complex and time-consuming, but would nevertheless be a routine undertaking for those of ordinary skill in the art having the benefit of this disclosure. It is furthermore to be readily understood that, although discussed below primarily within the context of providing right heart support during beating heart surgery, the cannulation system of the present invention may be employed in any number of cardiac procedures wherein the natural pumping ability of the heart needs to be augmented or replaced. The cannulation system disclosed herein boasts a variety of inventive features and components that warrant patent protection, both individually and in combination.

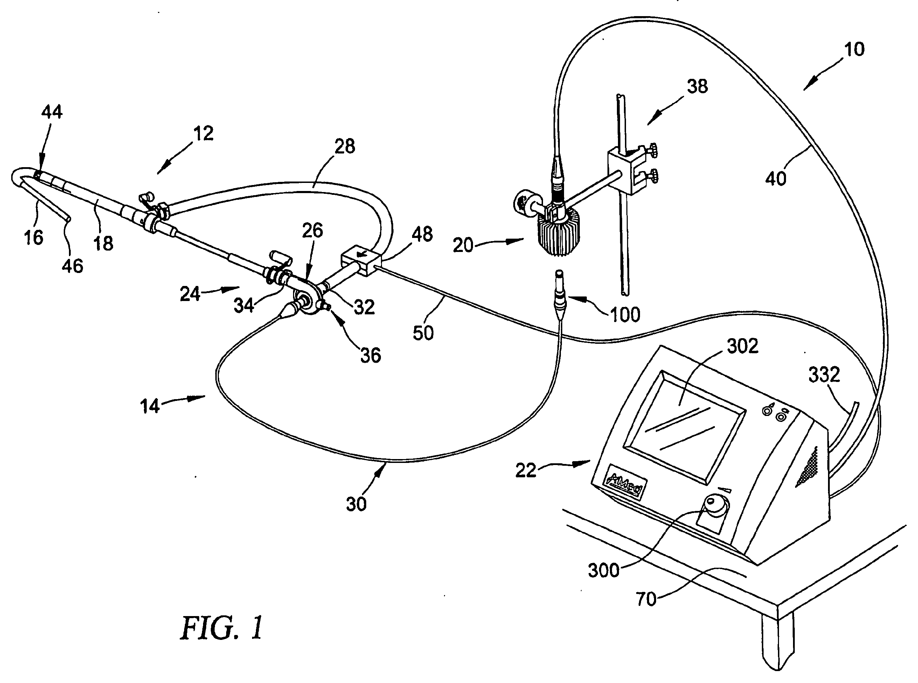

Referring initially to FIG. 1, shown in perspective is a cannulation system 10 according to a preferred embodiment of the present invention. The cannulation system 10 includes a coaxial cannula assembly 12 communicatively coupled to a centrifugal blood pumping system (referred to generally at 14). The coaxial cannula assembly 12 comprises an inner cannula 16 disposed generally concentrically through an outer cannula 18. The centrifugal blood pumping system 14 includes a motor 20, a control console 22, and a centrifugal pump assembly 24 comprising a miniature centrifugal blood pump 26, inflow tubing 28, and a magnetic drive cable assembly 30. The miniature centrifugal blood pump 26 has an inflow port 32 coupled to the outer cannula 18 (via inflow tubing 28), an outflow port 34 coupled to the inner cannula 16, and an internally disposed impeller assembly (not shown) coupled to the magnetic drive cable assembly 30. The blood pump 26 also includes a priming port 36 which, as will be described in greater detail below, is capable of being coupled to a syringe (not shown) for the purpose of evacuating air from within the pump 26 to prime the pump in preparation for use. The magnetic drive cable assembly 30 is dimensioned to be coupled to the motor 20. The motor 20, in turn, is communicatively linked to the control console 22 via an electrical control line 40.

Under the direction of the control console 22, the motor 20 may be operated such that the centrifugal pump assembly 24 selectively withdraws fluid (i.e. blood) through inflow apertures 42 formed in a basket portion 44 of the outer cannula 18, through a flow path defined between the interior surface of the lumen within the outer cannula 18 and the exterior surface of the inner cannula 16, and through the inflow tubing 28 before rerouting this fluid through the flow path defined within the lumen of the inner cannula 16 for delivery out a fluid outflow aperture 46 disposed at the distal end of the inner cannula 16. An optional flow probe 48 may be coupled to the inflow tubing 28 and communicatively linked to the control console 22 via control line 50 in order to aid in determining the flow rate produced by the centrifugal pump assembly 24. The magnetic drive cable assembly 30 provides the ability to position the miniature centrifugal pump 26 within the sterile field adjacent the operation site of the patient (not shown). In contrast to typical CPB circuits, this minimizes the distance the blood must travel before being reintroduced into the heart, thereby reducing the likelihood of hemolysis by decreasing the amount of foreign material that the blood is exposed to during operation. The motor 20 is preferably maintained adjacent yet outside the sterile field, such as through the use of a positioning assembly 38. The control console 22 may be disposed on any suitable structure disposed outside the sterile field, such as a table or cart 70.

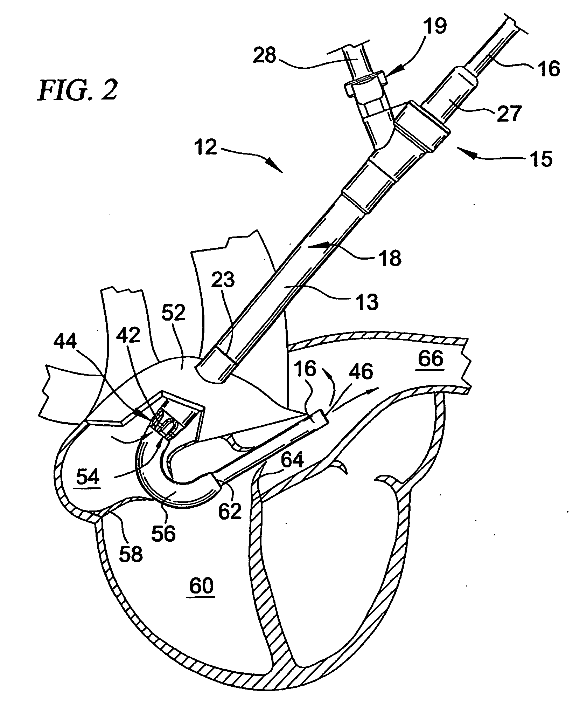

The coaxial cannula assembly 12 will now be described with reference to FIGS. 2-4. As shown in FIG. 2, the coaxial cannula assembly 12 of the present invention is particularly suited for use in providing right heart support during beating heart surgery. In this procedure, the outer cannula 18 is introduced into the heart through a single incision formed in the atrial appendage 52 such that the basket portion 44 is preferably disposed within the right atrium 54 and a curved J-tip portion 56 extends through the tricuspid valve 58 and into the right ventricle 60. In an important aspect, the J-tip portion 56 is generally rigid and curved such that the open distal end 62 thereof is directed generally at the pulmonary valve 64 when the basket portion 44 is positioned within the right atrium 54. In this fashion, the inner cannula 16 may be subsequently advanced through the outer cannula 18 and guided through the pulmonary valve 64 to position the fluid outflow aperture 46 of the inner cannula 16 within the pulmonary artery 66. The coaxial configuration of the cannula assembly 12 is advantageous in that it only requires a single incision to introduce both cannulas 16, 18 into the heart. The moveable relation between the inner cannula 16 and outer cannula 18 is advantageous in that it provides the ability to selectively position the blood outlet (i.e. the open distal end 46 of the inner cannula 16) relative to the blood inlet (i.e. the apertures 42 formed in the basket portion 44 of the outer cannula 18). As will be appreciated, this feature of adjustability provides the user with great latitude in employing the system of the present invention in patients having a wide variety of heart sizes.

The outer cannula 18 is constructed of a main body portion 13 having a Y-connector 15 on the proximal end and a J-tip portion 56 on the distal end. The main body portion 13 is preferably of wire-reinforced construction. The proximal end of the basket 44 is coupled to the distal end of the main body portion 13 of the outer cannula 18. The distal end of the basket portion 44 is coupled to the J-tip portion 56. In each instance, this coupling may be accomplished through a variety of techniques, including but not limited to the use of adhesives and/or ultrasonic welding. As used herein, the term "J-tip" is used to connote that the conduit forming the J-tip portion 56 has a curved shape similar to "J." It should be appreciated, however, that the exact curvature and shape of the portion 56 may be varied depending upon the application without departing from the scope of the present invention.

In a preferred embodiment (as will be described in greater detail below) the J-tip portion 56 may be coated with any of a variety of hydrogels such that, when moistened, the interior of the J-tip portion 56 includes a lubricious inner coating to assist the slideable advancement of the inner cannula 16 through the J-tip portion 56. The outer cannula 18 is preferably introduced into the heart of a patient such that the basket 44 is positioned in the right atrium while the J-tip portion 56 is located in the right ventricle. In an important aspect of the present invention, the distal opening 62 of the J-tip portion 56 is thus pointing generally in the direction of the pulmonic valve. The inner cannula 16, with the aid of the lubricious interior coating in the J-tip 56, may then be slideably advanced through the outer cannula 18 until the distal opening 46 is disposed a predetermined distance into the pulmonary artery. Depth marks 23, 25 are located on the wire reinforced main body portion 13 of the outer cannula 18 to indicate the preferred location of the basket 44 and J-tip portion 56 when the outer cannula 18 is introduced through the right atrial appendage.

The Y-connector 15 contains a hemostasis seal or port 17 and a side port having a female quick-connect fitting 19. The quick-connect side port 19 is a preferably a female fitting and has a tethered plug 21 for blocking the port 19 during insertion of the outer cannula 18 into a patient. The interior of the hemostasis seal 17 is dimensioned for hemostatic introduction of the inner cannula 16. In this fashion, the hemostasis port 17 serves to prevent fluid egress and ingress past the hemostasis port 17 along the exterior surface of the inner cannula 16. The exterior of the hemostasis seal 17 is dimensioned to serve as a port for removably receiving a docking member or cap 27 slideably disposed on the inner cannula 16. The placement of the docking cap 27 over the hemostasis port 17 further augments the hemostatic characteristics of the hemostasis port 17. As will be described in greater detail below, placing the docking cap 27 in this fashion also serves to anchor the inner cannula 16 relative to the outer cannula 18. This is particularly advantageous after the inner cannula 16 has been selectively advanced through the outer cannula 18 to place the open distal end 46 at a desired location within the pulmonary artery. The side port 19 of the Y-connector 15 is preferably disposed in the same general plane as the J-tip portion 56 such that the orientation of the J-tip portion 56 may be assessed when disposed within the heart based on the orientation of the side port 19 of the Y-connector 15. Other markings, such as one or more longitudinal lines, may be provided on the main body portion 13 of the outer cannula 18 to further assist in determining the orientation of the J-tip portion 56. The outer cannula 18 may be dimensioned in a variety of sizes, but is provided (by way of example only) having a 44 French outer diameter.

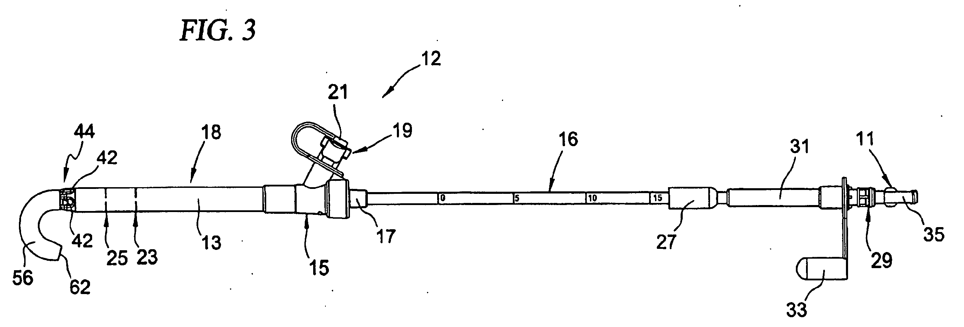

The inner cannula 16 is preferably constructed of flexible wire reinforced tubing. The proximal end terminates with a male quick-connect fitting 29 and a non-reinforced clamping section 31. As shown in FIG. 3, a stylet 11 is preferably preloaded within the inner cannula 16 to facilitate the insertion of the inner cannula 16 through the outer cannula 18, as well as to guide the cannula assembly 12 into the heart. The stylet 11 includes a handle portion 35 and a generally elongated portion (not shown) having a rigid distal section (not shown). The elongated portion extends through the interior of the inner cannula 16 and the rigid distal portion provides the ability to better advance the inner cannula 16 through the interior of the outer cannula 18. The rigid distal portion of the stylet 11 also is helpful in guiding the J-tip portion 56 of the outer cannula 18 into the right ventricle. That is to say, a user may wish to grip the Y-connector 15 of the outer cannula 18 and the handle portion 35 of the stylet 11 during insertion to help control the orientation of the J-tip portion 56. The proximal end of the stylet 11 is dimensioned to create a seal within the quick-connect fitting 29 of the inner cannula 16 during insertion.

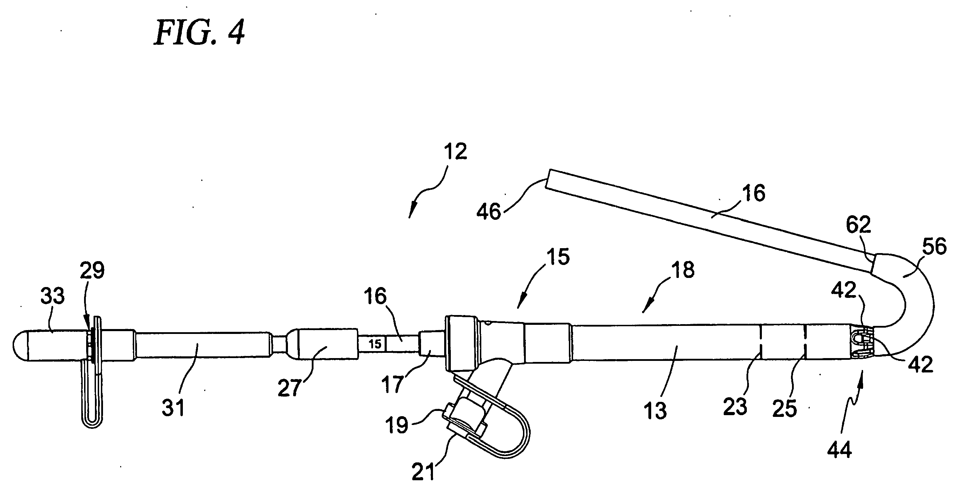

A tethered plug 33 is preferably coupled to the clamping section 31 for sealing or blocking the quick-connect fitting --29 after the stylet 11 has been removed (see FIG. 4). Depth markings (i.e. "0 cm," "5 cm," "10 cm," "15 cm, " etc...) are preferably provided on the wire reinforced section of the inner cannula 16 to indicate the distance between the distal tip 46 of the inner cannula 16 and the distal opening 62 of the J-tip portion 56. The docking member 27 is preferably loaded on the wire reinforced tubing at the proximal end of the inner cannula 16. Following insertion of the inner cannula 16 through the outer cannula 18, the docking member 27 may be moved into position over the hemostasis port 17 of the Y-connector 15 to secure and seals the inner cannula 16 within the hemostasis port 17. The inner cannula 16 may be dimensioned in a variety of sizes, but is provided (by way of example only) having a 22 French outer diameter.

As will be explained in greater detail below, the male quick-connect fitting 29 on the inner cannula is designed to engage with a corresponding female quick-connect fitting on the outlet 34 of the blood pump 26, while the female quick-connect fitting 19 on the outer cannula 18 is designed to engage with a corresponding male quick-connect fitting on the inlet tube 28 to the blood pump 26. The use of quick-connect fittings in this fashion is advantageous both in terms of safety and ease of use. Safety is served in that the inner cannula 16 and outer cannula 18 may only be coupled to the centrifugal blood pump assembly 24 in the manner designed due to the use of one male and one female quick-connect fitting on the pump assembly 24. In this fashion, the possibility of hooking up the system incorrectly (such as with the inner cannula 16 coupled to the inflow port 32 of the pump assembly 24 or the outer cannula 18 coupled to the outflow port 34 of the pump assembly 24) is altogether eliminated. Moreover, an audible click will be heard (and tactile "snap") when the quick-connect fittings are correctly latched, thereby providing a further measure of safety. Ease of use is served in that a physician need only "snap" the mating quick-connect fittings together to establish the necessary connection to the extracorporeal circulation circuit. This is far faster and thus superior to the prior art technique for coupling cannulas to blood pumps which merely involves press-fitting the open end of a cannula over a barb-fitting on the inlet or outlet of the blood pump. This prior art technique is also disadvantageous in that the barb-fitting couplings are more difficult to remove. This may be especially important where time is critical, such as where a pump needs to be quickly changed out during a procedure. To disconnect the quick-connect fittings of the present invention, a user need only depress the latch with the thumb and pull the fittings apart. The quick-connect fittings of the present invention thus provide the ability to quickly remove the inner cannula 16 and outer cannula 18 from the pump assembly 24, representing a significant improvement over the prior art. The quick-connect fittings referenced herein may comprise any number of different types of mating male/female quick- connect couplings, including but not limited to the type shown and described in U.S. Patent No. 5,052,725.

Further explanation of the coaxial cannula assembly 12 may also be found with reference to commonly assigned and co-pending U.S. Patent Application Ser. No. 09/481,730 entitled "Methods and Systems for Right and/or Left Heart Support During Cardiac Surgery," filed January 11, 2000, and Int'1 Patent Application Ser. No. PCT/US99/1366 entitled "Apparatus and Methods for Entering Cavities of the Body," filed June 18, 1999.

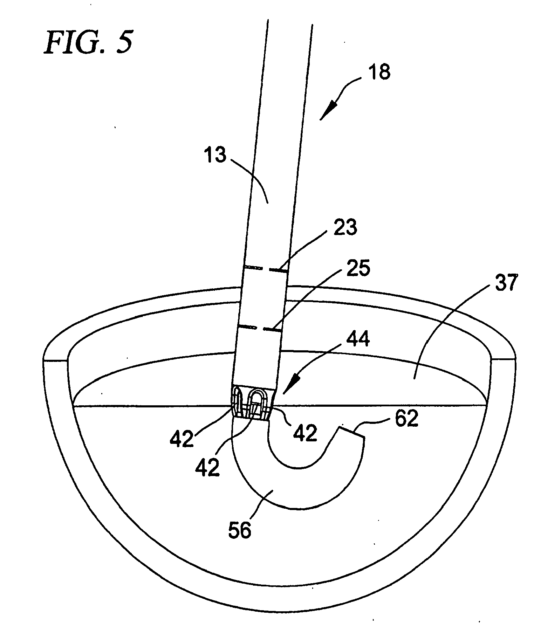

A method of assembling and using the coaxial cannula assembly 12 will now be described. First, the coaxial cannula assembly 12 must be prepared for introduction into a patient's heart. This involves preloading the inner cannula 16 within the outer cannula 18 as shown in FIG. 3. To do so, the inner cannula 16 is preferably first preloaded with a stylet 11 to add rigidity to the inner cannula 16 during introduction into the outer cannula 18. Preloading the stylet 11 in this fashion creates a seal between the interior of the quick-connect fitting 29 and the exterior of the stylet 11, thereby sealing off the interior of the inner cannula 16. The inner cannula 16 is introduced through the Y-connector 15 and into the outer cannula 18 such that the distal end 46 of the inner cannula 16 is disposed in the approximate vicinity of the inflow basket 44. The interior of the outer cannula 18 is sealed off by seating the plug 21 within the side port quick-connect fitting 19 of the Y-connector 15. Next, the outer cannula 18 must be dipped in a sterile physiologic solution as shown in FIG. 5 to moisten the hydrogel coating on the J-tip portion 56. Dipping the outer cannula 18 in this fashion causes the hydrogel coating on the J-tip portion 56 to absorb moisture and become lubricious to ease the advancement of the inner cannula 16.

The inner cannula 16 must then be advanced through the outer cannula 18 until its open distal tip 46 extends a short distance (preferably 1 cm) beyond the distal opening 62 of the J-tip portion 56. To do so, the stylet 11 must first be withdrawn (either partially or fully). This is because the distal end of the stylet 11 is rigid and thus prevents the distal end 46 from bending through the J-tip portion 56. If the stylet 11 is fully removed, the tethered cap 33 must be placed over the male quick-connect fitting 29. In a preferred embodiment, the inner cannula 16 is equipped with markings (0 cm, 5 cm, 10 cm, 15 cm, etc) which, when aligned with the approximate end of the hemostasis seal 17, indicate the distance the distal tip 46 of the inner cannula 16 extends past the end 62 of the J-tip portion 56. As such, the task of positioning the distal end 46 of the inner cannula 16 a short distance beyond the distal opening 62 of the J-tip portion 56 may be easily accomplished by advancing the inner cannula 16 such that "0 cm" mark is positioned at the approximate end of (or slightly inside) the hemostasis port 17 of the Y-connector 15. The docking cap 27 on the inner cannula 16 should preferably be positioned between the "15 cm" mark and the non-reinforced clamping portion 31 of the inner cannula 16 during the advancement of the inner cannula 16 through the outer cannula 18.

The coaxial cannula assembly 12 must next be introduced into the patient's heart. When used for right heart support, an incision should be created in the right atrial appendage with a purse-string suture established thereabout in a known fashion. The Y-connector 15 of the outer cannula 18 should then preferably be held with the side-port 19 of the Y-connector 15 pointing up and the proximal end of the cannula toward the patient's feet. The lateral edge of the atriotomy should then be secured with a forcep to provide counter-traction during insertion. The coaxial cannula assembly 12 should then be partially inserted such that the distal tip 46 of the inner cannula 16 and the distal tip 62 of J-tip portion 56 are disposed in the atriotomy. The vascular clamp on the purse-string suture should then be released and the J-tip portion 56 partially inserted therethrough while providing slight tension on the purse-string to control leakage. The outer cannula 18 should then be gently advanced into the heart using a counter-clockwise rotating motion such that the J-tip portion 56 advances across the tricuspid valve and points generally in the direction of the pulmonic valve. At this point, the basket 44 of the outer cannula 18 should automatically be positioned inside the right atrium. This automatic positioning may be facilitated through the use of markings 23, 25 on the main body portion 13 of the outer cannula 18. Markings 23, 25 are designed to ensure the proper position of the basket portion 44 and J-tip portion 56 when the atrial wall is disposed between the marks 23, 25. Once inserted, the purse string should be tightened to establish a tourniquet around the cannula body 13. The location of the J-tip portion 56 within the right ventricle should preferably be verified, such as by palpating the lateral wall of the inferior vena cava and/or right ventricle. Doing so will allow a person to feel the curve of the J-tip portion 56 passing through the tricuspid valve.

The inner cannula 16 should then be advanced through the outer cannula 18 until the open distal tip of the inner cannula 16 is disposed in the pulmonary artery. Before doing so, the stylet 11 should be removed (if it hasn't been already) and the cap 33 disposed over the quick-connect fitting 29 of the inner cannula 16. The stylet 11 must be so removed because the inner cannula 16 will not track through the J-tip portion 56 with the stylet 11 in place. The inner cannula 16 should then be gently advanced through right ventricular outflow tract and into the pulmonary artery. The preferred location of the distal tip of the inner cannula 16 is proximal to the pulmonary artery bifurcation. Typically, this occurs when the "15 cm" mark on the inner cannula 16 is approximately aligned with the rim of the hemostasis port 17, indicating that the open distal end of the inner cannula 16 is disposed approximately 15 cm from the distal opening of the J-tip portion 56 (see FIGS. 2 and 4). The location of the inner cannula 16 within the pulmonary artery should be verified, such as by palpating the pulmonary artery. The location of the open distal tip of the inner cannula 16 may preferably be adjusted if not in the desired location. To do so, the docking cap 27 should be temporarily disengaged and/or the outer cannula 18 repositioned before gently advancing the inner cannula 16. Following any adjustments, the docking cap 27 should be advanced along the inner cannula 16 (while holding the proximal end of the inner cannula 16 to prevent movement) until it docks over the hemostasis port 17 of the Y-connector 15. The docking cap 27 thus secures the location of the inner cannula 16 relative to the outer cannula 18 and also provides a secondary hemostasis seal above and beyond the hemostasis seal (not shown) disposed within the Y-connector 15. The location of the tip 46 of the inner cannula 16 may be adjusted while the docking cap 27 is secured to the hemostasis port 17. However, the force required to move the inner cannula 16 must necessarily be greater than when the docking cap 27 is removed from the hemostasis port 17. Any such adjustment should occur before coupling the coaxial cannula assembly 12 to an extracorporeal circulation system (such as the centrifugal pumping assembly described herein) so as to avoid leakage.

The coaxial cannula assembly 12 of the present invention may then be coupled to an extracorporeal circulation system, such as the centrifugal blood pumping system 14 to be explained in greater detail below. Before doing so, the non-reinforced clamping portion 31 of the inner cannula 16 must be clamped such that the cap 33 may be removed from the quick-connect fitting 29 without leakage. At this point, the female quick-connect fitting 19 on the Y-connector 15 may be easily coupled to a corresponding male quick-connect fitting on the inlet tubing 28 of the centrifugal blood pump 26. The male quick-connect fitting 29 on the inner cannula 16 may be similarly coupled to a corresponding female quick-connect fitting on the outflow port 34 of the centrifugal blood pump 26. As detailed above, the use of such male/female quick-connect fittings is advantageous in terms of both safety and ease-of-use.

The centrifugal blood pump assembly 24 must be deaired before use. While the specifics of the blood pump assembly 24 will be set forth in greater detail below, an important feature to note now is the priming port 36 on the pump 26. The priming port 36 provides the ability to perform this de-airing step quickly and easily by simply attaching a syringe (not shown) to the priming port 36 and withdrawing the plunger to remove the air disposed within the pump assembly 24. In a preferred embodiment (set forth by way of example only), the priming volume of the pump 26 is approximately 12 ml, while the priming volume of the pump assembly 24 (pump 26 and inlet tubing 28) is approximately 33 ml. It is to be readily appreciated, however, that these priming volumes will vary depending upon the dimensions of the pump 26 and inlet tubing 28 and that such variations are within the scope of the present invention.

With the system primed, the centrifugal blood pumping system 14 may then be operated to withdraw blood from the right atrium and redirect it into the pulmonary artery. To do so, the magnetic drive cable assembly 30 is first coupled to the motor 20. The motor 20 must be communicatively linked to the control console 22, such as via the electrical control line 40. As will be explained in greater detail below, the control console 22 may then be operated such that the centrifugal pump assembly 24 selectively withdraws blood from the right atrium (via inlet ports 42 in the outer cannula 18) for redeposit in the pulmonary artery (via the open distal end 46 of the inner cannula 16). The operation of the control console 22 may be based on a host of variables or characteristics, including but not limited to motor speed, motor current, motor voltage, battery voltage, flow rate, blood pressure, and cardiac output. The speed of the motor 20 and hence pump 26 may be controlled to provide a wide variety of flow rates. It is preferred to maintain flow rates sufficient to maintain the cardiac output of the patient at stable levels throughout the beating heart surgery.

Following use, the cannula assembly 12 must be withdrawn from the patient. To do so, the clamping section 31 of the inner cannula 16 should be clamped while generally simultaneously discontinuing extracorporeal circulation, such as by stopping or slowing the pump 26. The inner cannula 16 should then be retracted within the outer cannula 18. Preferably, this will be performed such that the distal tip 46 of the inner cannula 16 will be disposed within the main body portion 13 of the outer cannula 18. This will provide the distal tip 46 of the inner cannula 16 in communication with right atrial pressure instead of right ventricular pressures, thereby aiding the return of blood into the heart. Positioning the distal end 46 in this fashion may be accomplished by withdrawing the inner cannula 16 until the "0 cm" mark is disposed approximately 5 cm past (out from) the hemostasis port 17 of the Y-connector 15. The inner cannula 16 may then be disconnected from the pump assembly 24 by depressing the latch of the female quick-connect fitting on the outlet 34 of the pump 26 and pulling the inner cannula 16 out of the now-unlatched fitting. The prime in the outer cannula 18 must be returned to the right atrium, such as by elevating the pumping circuit connected to the outer cannula 18. The prime in the inner cannula 16 must similarly be returned to the right atrium, such as by elevating the inner cannula 16. The cannula assembly 12 may then be removed from the heart by rotating the axis of the outer cannula 18 in a clockwise motion towards the patient's feet while retracting the cannula 12 from the atriotomy. The atriotomy must then be closed in a known fashion. The cannula assembly 12, stylet 11, and blood pump assembly 24 should then be disposed of according to hospital procedures for handling contaminated materials.

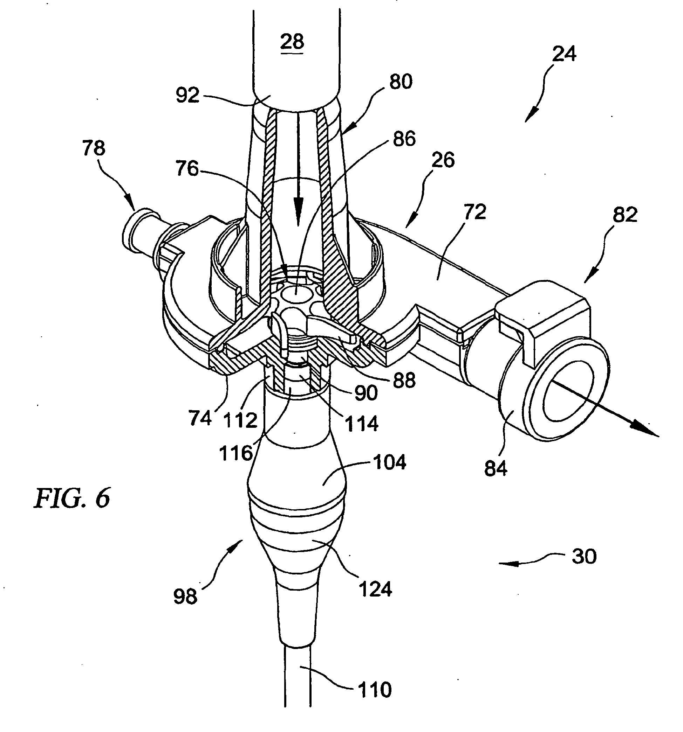

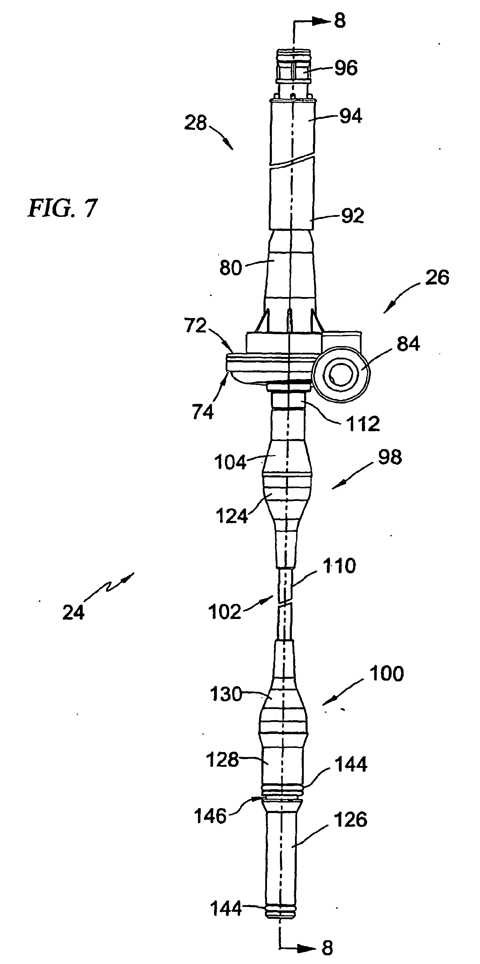

The centrifugal pump assembly 24 will now be described in detail with reference to FIGS. 6-8. The centrifugal pump assembly 24 includes the miniature centrifugal blood pump 26, inflow tubing 28, and magnetic drive cable assembly 30. The miniature centrifugal blood pump 26 comprises first and second pump housing members 72, 74 (which collectively form a pump housing assembly), an impeller assembly 76, a priming port 78, a fluid inlet port 80 forming part of the first pump housing member 72, and a fluid outlet port 82 equipped with a female quick-connect fitting 84. As will be explained in greater detail below, the impeller assembly 76 comprises an impeller 86 coupled to a shaft 90. The impeller 86 includes a plurality of blades 88 extending generally radially therefrom. The shaft 90 extends generally perpendicularly from the impeller 86 for connection to the magnetic drive cable assembly 30. The priming port 78 provides the ability to communicatively couple a de-airing device, such as the syringe referenced above, with the interior of the pump housing assembly for the purpose of priming the centrifugal blood pump 26 before use. In the embodiment shown, the priming port 78 comprises a luer-type fitting commonly known in the art for coupling with syringes. It is to be readily understood that any number of alternate coupling arrangements may be provided for coupling de-airing devices to the pump 26 without departing from the scope of the present invention. The female quick-connect fitting 84 and the luer-type priming port 78 are rigidly bonded to the pump housing, such as through the combined use of adhesives and ultraviolet welding techniques. Providing the priming port 78 in this manner is advantageous in that it provides a quick, easy, and convenient fashion to couple and decouple priming devices to and from the pump 26. Providing the female quick-connect fitting 84 in this manner is advantageous in that it provides a quick, easy and convenient fashion to couple and de-couple the fluid outlet 34 of the pump 26 to and from the male quick-connect fitting 29 on the inner cannula 16.

The inflow tubing 28 comprises a generally flexible length of plastic conduit having a first end 92 bonded to the fluid inlet port 80 of the first pump housing member 72 and a second end 94 bonded to a male quick-connect fitting 96. As above, these bonds may be achieved through the combined use of adhesives and ultraviolet welding techniques. The male quick-connect fitting 96 is dimensioned to cooperatively couple with a female quick-connect fitting provided as part of the outer cannula 18 discussed above. Equipping the inflow tubing 28 with the male quick-connect fitting 96 is advantageous in that it provides a quick, easy, and convenient fashion to couple and de-couple the fluid inlet port 80 of the pump 26 to and from the female quick-connect fitting 19 of the outer cannula 18. The inflow tubing 28 may comprise any number of types of tubing of varying dimensions. In a preferred embodiment, the inflow tubing 28 is 0,95 cm x 1,43 cm (3/8" x 9/16") Tygon® tubing having a length of approximately 33 cm (13 inches).

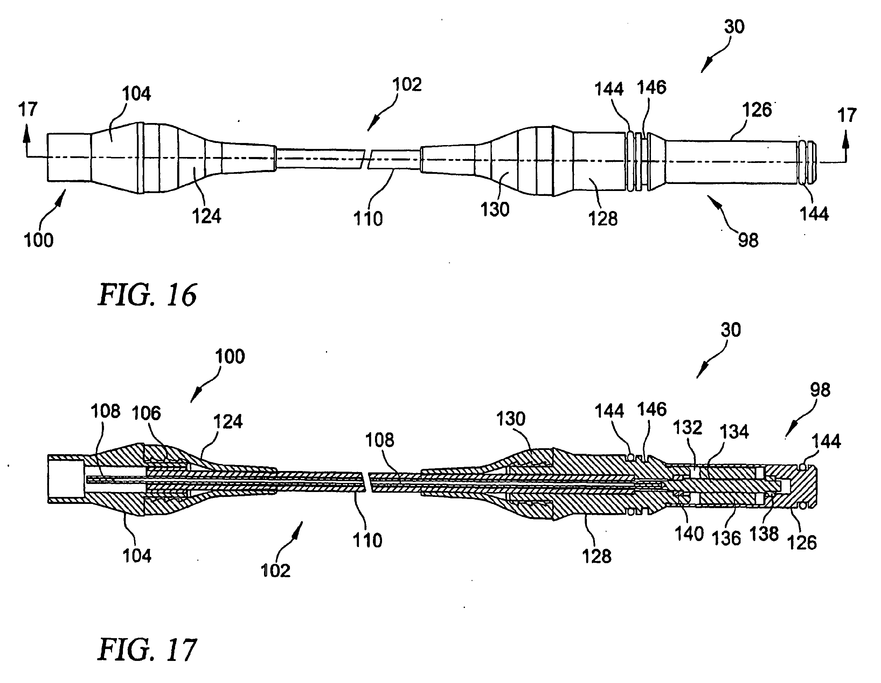

The magnetic drive cable assembly 30 of the present invention will now be described with combined reference to FIGS. 6-8 and FIGS. 16-17. Magnetic drive cable assembly 30 includes a pump coupling assembly 98, a motor coupling assembly 100, and a sheathed drive cable assembly 102 extending therebetween. The sheathed drive cable assembly 102 includes a drive cable 108 rotatably disposed within a generally flexible sheath or tubing member 110. The pump coupling assembly 98 includes a bearing housing cap 104 rigidly coupled to a portion 112 of the second housing member 74 which houses various bearing components associated with the shaft 90. These bearing components include a bearing 114, a spacer 116, a flanged bearing 118, and a retention ring 120. A seal 121 is also provided disposed about the shaft 90 near its connection to the impeller 86. The bearing housing cap 104 includes a sheath retainer 106 for fixedly receiving the sheath 110 of the drive cable assembly 102. The drive cable 108 extends through a lumen formed through the housing cap 104 for connection to the shaft 90. The pump coupling assembly 98 also includes a strain relief member 124 disposed about the sheath retainer 106 and an adjacent portion of the sheathed drive cable assembly 102.

The motor coupling assembly 100 includes a magnet housing 126, a magnet housing cap 128, and a strain relief member 130. The magnet housing 126 is dimensioned to receive a magnet shaft assembly 132 comprising a shaft 134 fixedly coupled to the drive cable 108 and a magnet 136. The magnet shaft assembly 132 is rotatably disposed within the motor coupling assembly 100 through the use of a bearing 138 in the magnet housing 126 and a bearing 140 in the magnet housing cap 128. The magnet housing cap 128 includes a sheath retainer 142 and a lumen dimensioned to fixedly receive the sheath 110 of the sheathed drive cable assembly 102 therewithin. In this fashion, the drive cable 108 and magnetic shaft assembly 132 are hermetically sealed from the pumping chamber of the pump 26. The magnet housing 126 and the magnet housing cap 128 are dimensioned to be quickly and easily coupled to a motor (such as the motor 20 in FIGS. 1 and 3) having a magnetic rotor capable of magnetically driving the magnet shaft assembly 132 into rotation. 0-rings 144 and an engagement groove 146 may be provided to facilitate the quick and convenient coupling/de-coupling feature of the magnetic drive cable assembly 30 of the present invention. Under the direction of the magnetic rotor, the drive cable 108 will be forced into rotation within the sheath 110 to thereby cause the impeller assembly 76 to rotate within the pump 26. As will be discussed in greater detail below, the impeller assembly 76 is configured to rotate in a clockwise fashion within the pump 26 such that fluid will be drawn into the fluid inlet port 78 (after passing through the inlet tubing 28) and delivered in a generally tangential fashion out the fluid outlet port 82.

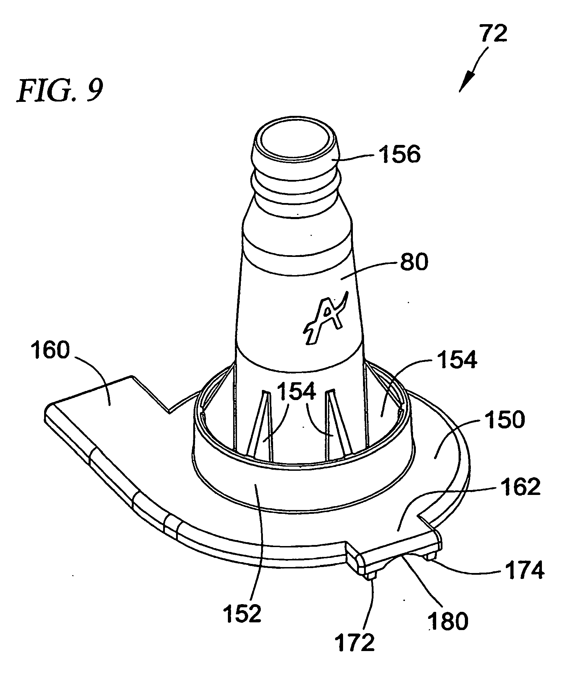

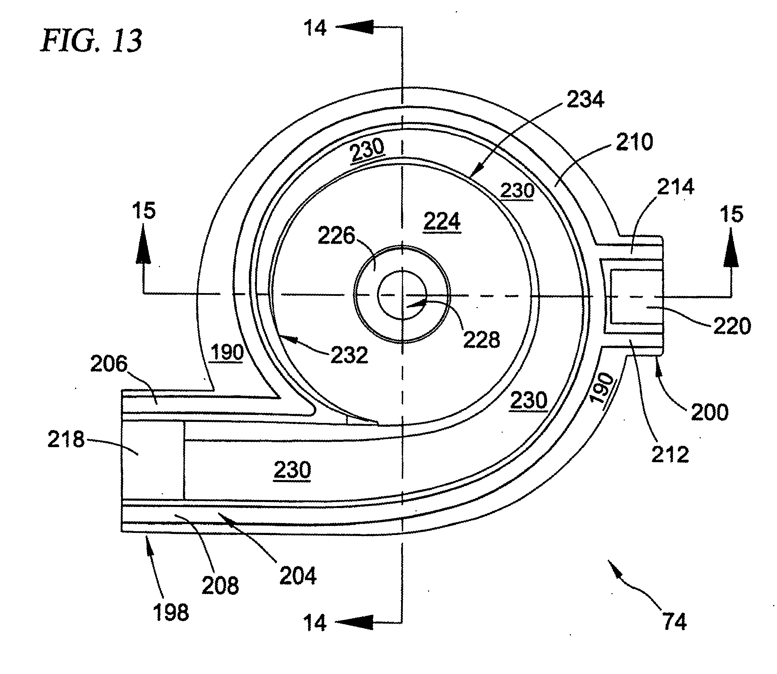

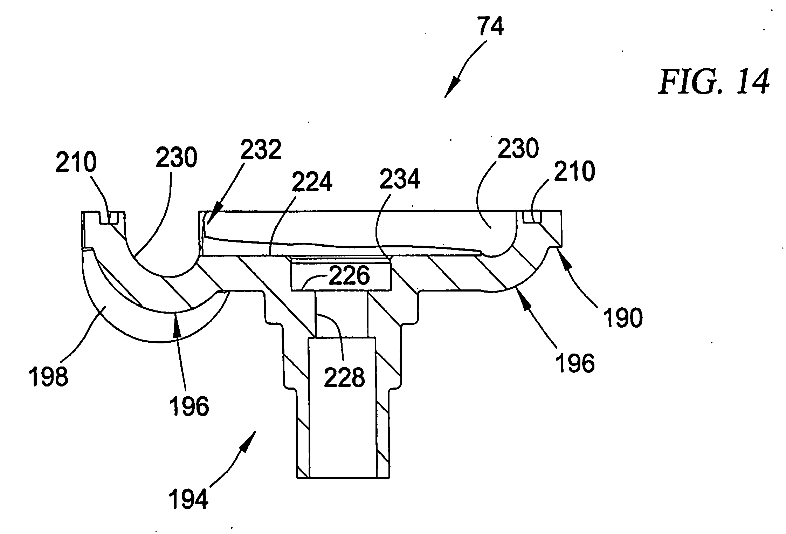

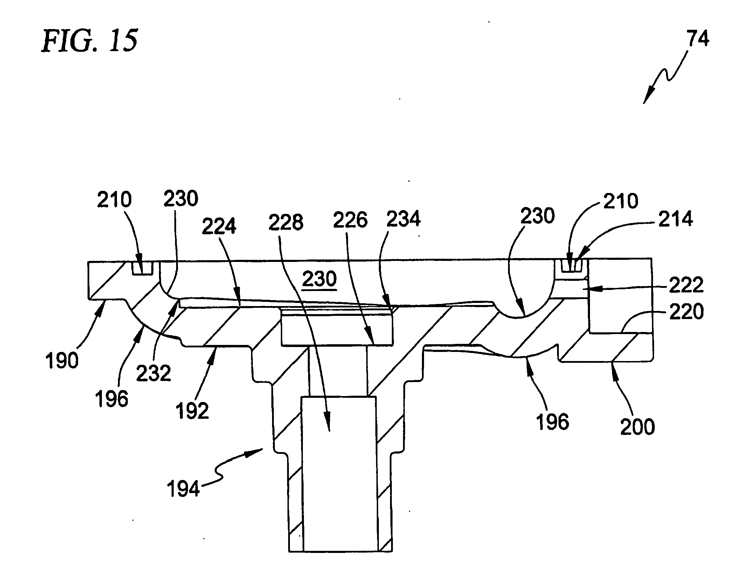

The miniature centrifugal pump 26 of the present invention will now be further described in detail with reference to FIGS. 9-15. Referring initially to FIGS. 9-10, the first pump housing member 72 is preferably a molded component of unitary construction having a generally planar base portion 150 from which the fluid inlet port 80 extends in a generally perpendicular fashion. An annular ridge 152 is provided extending generally perpendicularly from the base portion 150 about the base of the fluid inlet port 80. A plurality of buttress members 154 extend between the annular ridge 152 and the base of the fluid inlet port 80. A plurality of engagement ridges 156 are provided on the fluid inlet port 80 adjacent its open distal end to facilitate coupling the fluid inlet port 80 with the inflow tubing 28. This coupling may be augmented through various well-known techniques, including but not limiting to the use of adhesives and/or ultrasonic welding. The base portion 150 includes a first extending section 160 which, as will be described in further detail below, matingly engages with a corresponding portion of the second pump housing member 74 to define an aperture within which the female quick-connect fitting 84 may be bonded according to the present invention. The base portion 150 includes a second extending section 162 which, as will also be described in greater detail below, matingly engages with a corresponding portion of the second pump housing member 74 to define an aperture within which the luer-type priming port 36 may be bonded according to the present invention.

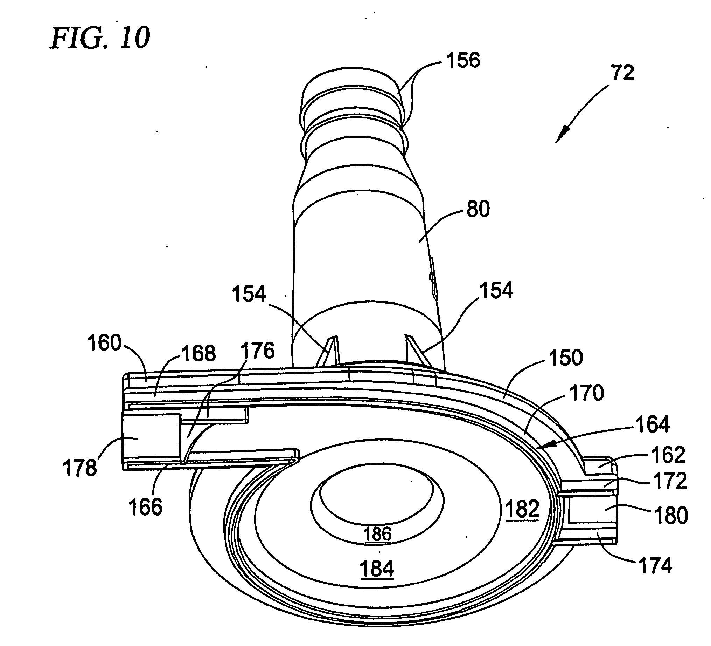

With reference to FIG. 10, the mating engagement of the first pump housing member 72 to the second pump housing member 74 is facilitated by molding an engagement ridge 164 as part of the first pump housing member 72 that extends generally perpendicularly from the interior surface of the base portion 150. As will be set forth in greater detail below, the engagement ridge 164 of the first pump housing member 72 is dimensioned to matingly cooperate with a corresponding engagement groove formed in the second pump housing member 74 to facilitate bonding the first and second pump housing members 72, 74 together during the manufacture of the miniature centrifugal blood pump 26 of the present invention. The engagement ridge 164 includes a first generally straight portion 166 and a second generally straight portion 168 which extend inward from the section 160 of the base member 150 for connection to a generally spiral portion 170. In a preferred embodiment, the generally spiral portion 170 has an expanding radius as it extends from the first generally straight portion 166 for connection to the second generally straight portion 168. The engagement ridge 164 also includes third and fourth generally straight portions 172, 174 which extend inward from the section 162 of the base member 150 for connection to the generally spiral portion 170. An abutment member 176 is disposed in between the first and second generally straight portions 166, 168 of the engagement ridge 164. The abutment member 176 helps define a seat against which the female quick-connect fitting 84 is brought to rest to ensure the fitting 84 is properly registered when it is bonded to the pump housing members 72, 74 during manufacture. A curved notch 178 may be formed on the underside of the section 160 of base 150 adjacent the abutment member 176 to conform to the contour of the portion of the female quick-connect fitting 84 that extends into the pump housing. In similar fashion, a curved notch 180 may be formed on the underside of the section 162 of base 150 to conform to the contour of the portion of the luer-type priming port 78 that extends into the pump housing. The underside of the base 150 is also characterized as including a generally planar surface 182 which has a width that expands radially corresponding to the radial expansion of the generally spiral portion 170 of the engagement ridge 164 along the base 150. Within this generally planar surface 182 are progressively curved surfaces 184, 186 which provide a smooth transition into the lumen defined within the fluid inlet port 80.

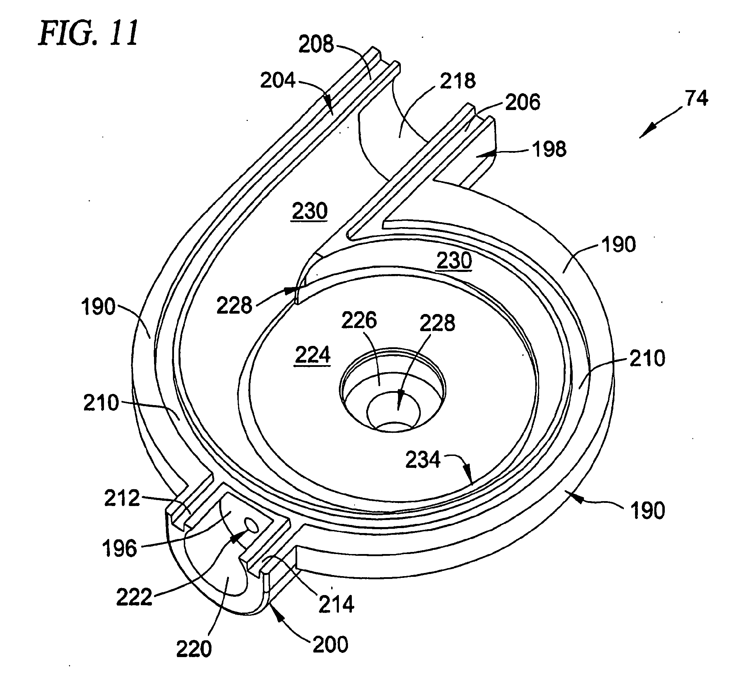

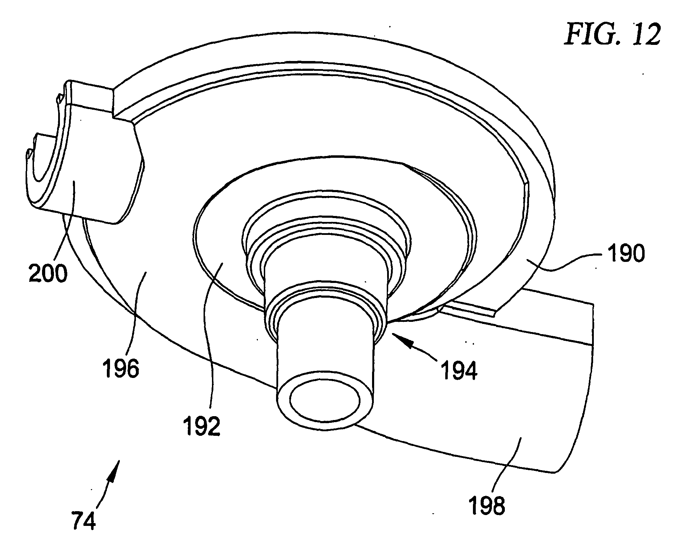

The second pump housing member 74 of the present invention will now be described in detail with reference to FIGS. 11-15. Referring initially to FIG. 12, the second pump housing member 74 is preferably a molded component of unitary construction. The pump housing member 74 includes a generally planar base portion 190, a generally planar central portion 192 disposed in a generally raised and parallel fashion relative to the base portion 190, and a tiered bearing housing 194 extending generally perpendicularly from the central portion 192. A generally spiral volute portion 196 is also provided having a radially expanding width and a vertically increasing height as the volute portion 196 progresses counter-clockwise (as viewed in FIG. 12) along the base 190. The volute portion 196 includes a generally straight, partially tubular section 198 that extends outward from the base portion 190. When the first and second pump housing members 72, 74 are coupled together, the generally straight section 198 cooperates with the first extending section 160 to define an aperture within which the female quick-connect fitting 84 is bonded according to the present invention. A generally straight, partially tubular portion 200 is also provided extending from the volute portion 196. During manufacture of the pump 26, the portion 200 cooperates with the second extending section 162 of the first pump housing member 72 to define an aperture within which a coupling mechanism (such as the luer-type priming port 78 shown above) may be bonded according to the present invention. As will be set forth in greater detail below, an air-evacuation aperture (not shown) is formed through the wall of the volute portion 196 within the tubular portion 200 such the coupling mechanism bonded therewithin may be employed with an air-evacuation device (such as the syringe 38 discussed above) to remove air from within the pump 26 to prime the pump 26 before use.

With reference to FIGS. 11 and 13-15, the interior surface of the second pump housing member 74 is equipped with an engagement groove 204 dimensioned to matingly receive the engagement ridge 164 of the first pump housing member 72 such that the first and second pump housing members 72, 74 may be bonded together during the manufacture of the miniature centrifugal pump 26 of the present invention. The engagement groove 204 includes a first straight portion 206 and a second straight portion 208 which extend inward from the generally tubular portion 198 for connection to a generally spiral portion 210. In a preferred embodiment, the generally spiral portion 210 has an expanding radius as it extends from the first generally straight portion 206 for connection to the second generally straight portion 208. The engagement groove 204 also includes third and fourth generally straight portions 212, 214 which extend inward from the partially tubular portion 200 for connection to the generally spiral portion 210. As will be appreciated, the portions 206-214 of the engagement groove 204 correspond to the portions 166-174 of the engagement ridge 164 discussed above. A curved notch 218 may be formed on the underside of the generally straight, partially tubular portion 198 to conform to the contour of the portion of the female quick-connect fitting 84 that extends into the pump housing. In similar fashion, a curved inner surface 220 of the tubular portion 200 conforms to the contour of the portion of a coupling mechanism (such as the luer-type priming port 78 disclosed above) so as to ensure proper registry during the bonding process. An air-evacuation port 222 is formed through the wall of the volute portion 196 to provide fluid communication between the coupling mechanism (i.e. priming port 78) and the interior of the pump 26 for priming purposes.

The interior of the second pump housing member 74 is also characterized as including a central region 224 having a generally planar surface corresponding to the central portion 192 discussed above with regard to the exterior of the second pump housing member 74. An annular seat 226 is provided within the central region 224 for receiving the rotor seal 121 discussed above. A shaft aperture 228 extends through the annular seat 226 such that the shaft 90 of the impeller assembly 76 may be passed therethrough for connection to the drive cable 108 of the present invention. As will be explained in greater detail below, the blades 88 of the impeller 86 are positioned generally within the central region 224 when the shaft 90 is passed through the shaft aperture 228. In an important aspect of the present invention, the impeller 86 cooperates with a volute 230 formed along the interior of the volute portion 196 discussed above to provide enhanced fluid pumping characteristics, such as increased fluid pressure for a given pump speed, and decreased hemolysis when pumping blood.

The volute 230 of the present invention has an origin (designated generally at 232) located a vertical distance above the surface of the central region 224. As it progresses in a clockwise fashion away from the origin 232, the shape of the volute 230 expands in both a vertically downward direction and a radially outward direction. In a preferred embodiment of the present invention, the rate of vertical expansion is approximately 2.5 degrees as the volute 230 progresses away from the origin 232, and the rate of radial expansion is approximately 5 degrees as the volute 230 progresses away from the origin 232. In this fashion, the volute 230 starts out above the surface of the central region 224 and then drops below the surface of the central region 224 at a location denoted generally at 234 and keeps expanding in a vertically downward fashion until approximately the beginning of the curved notch 218. It is to be readily understood that the scale and size of the first and second pump housing members 72, 74 may be selected and/or adjusted over a wide range such that the centrifugal pump 26 of the present invention may be dimensioned to be suitable for use in a wide variety of fluid pumping applications. For example, the rate of vertical and/or radial expansion of the volute 230 may be anywhere in the range of between 1 and 15 degrees without departing from the scope of the invention.

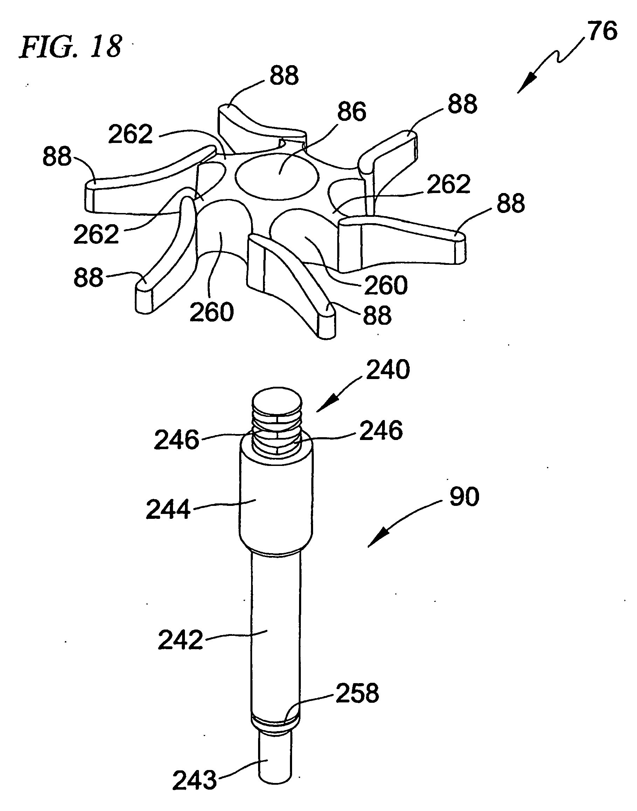

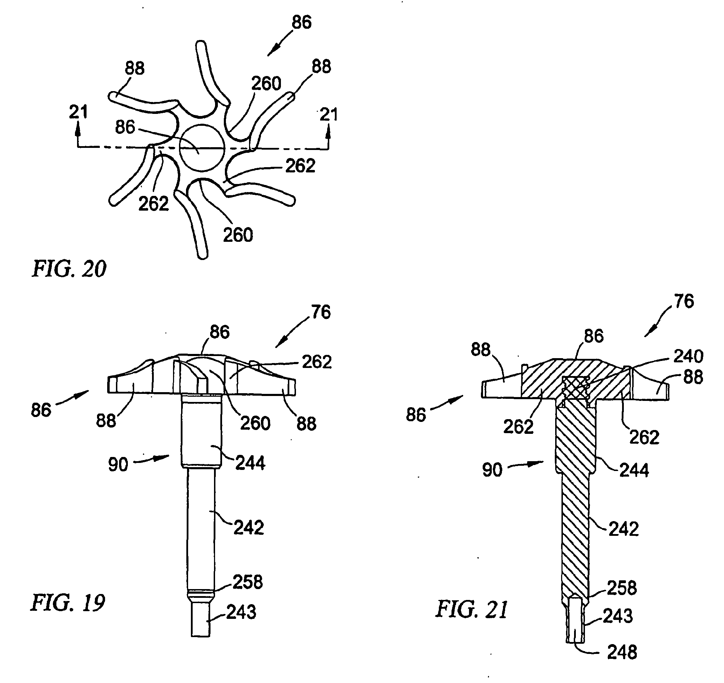

The impeller assembly 76 of the present invention will now be discussed in detail with reference to FIGS. 18-21. The impeller assembly 76 includes the shaft 90 rigidly coupled to the impeller 86, which is equipped with a plurality of rotor blades 88 for directing fluid within the pump 26. The shaft 90 may be constructed of any number of suitable materials, including but not limited to stainless steel. The impeller 86 and blades 88 may be constructed from any number of suitable materials, including but not limited to thermoplastics such as polycarbonate. The shaft 90 of the impeller assembly 76 is a generally cylindrical member of unibody construction. As shown most clearly in FIG. 18, the shaft 90 includes an impeller coupling portion 240, an upper cylindrical upper portion 244, a lower cylindrical portion 243, and an intermediate cylindrical portion 242. The impeller coupling portion 240 includes a plurality of intersecting threads 246 which, after the impeller 86 is molded thereabout during manufacture, serve to prevent the impeller 86 from unscrewing or becoming dislodged due to the rotational forces experienced during use. As shown in FIG. 21, the lower portion 243 of the shaft 90 is generally hollow having an internal lumen 248 for matingly receiving the drive cable 108 of the magnetic drive cable assembly 30. The drive cable 108 is preferably fixed to the shaft 90 by crimping the drive cable 108 within internal lumen 248 of the lower portion 243. The impeller 86 of the impeller assembly 76 is preferably constructed having a plurality of cut-out portions 260. The cut-out portions 260 define a plurality of bridging ribs 262 that extend radially away from the center of the impeller 86 for connection with the leading edge of a respective blade member 88.

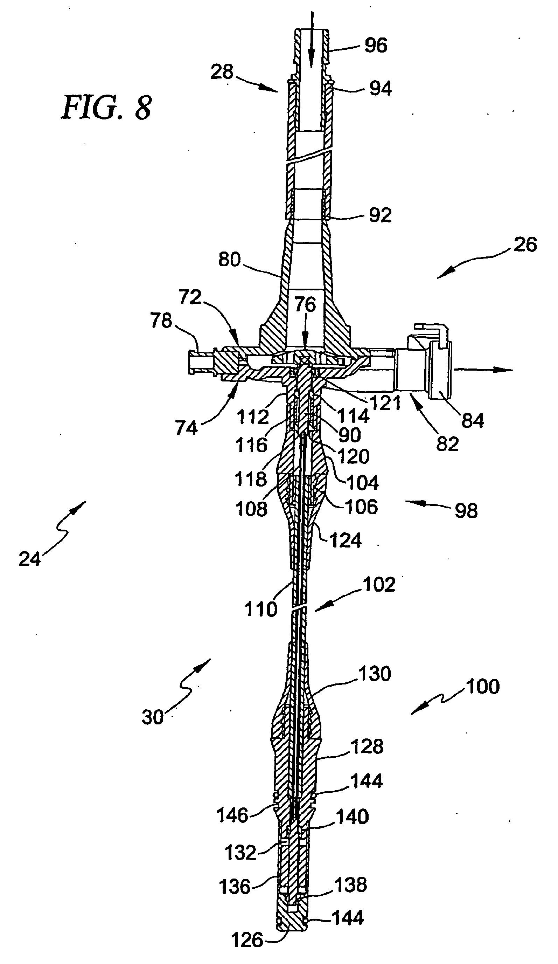

As shown in FIGS. 6 and 8, the impeller assembly 76 of the present invention is disposed within the pump 26 such that the impeller 86 is located in between the first and second pump housing members 72, 74. with further reference to FIGS. 11-15, the shaft 90 is dimensioned to extend through the shaft aperture 228 and bearing housing 194 of the second pump housing member 74. In this arrangement, the upper portion 244 of the shaft 90 has the seal 121 disposed about its proximal end and the bearing 114 bounding its distal end. The lower portion 242 extends distally away from the upper portion 244 and is supported within the bearing housing 194 by the bearing 114, the spacer 116, and the flanged bearing 118. The retention ring 120 is provided to matingly engage within a groove 258 formed on the intermediate portion 242 of the shaft 90 for the purpose of maintaining the seals 114, 118 and spacer 116 in position around the lower portion 242. This allows the shaft 90 to rotate within the rotor bearing housing 194 under the direction of the motor 20 coupled to the magnetic drive cable assembly 30.

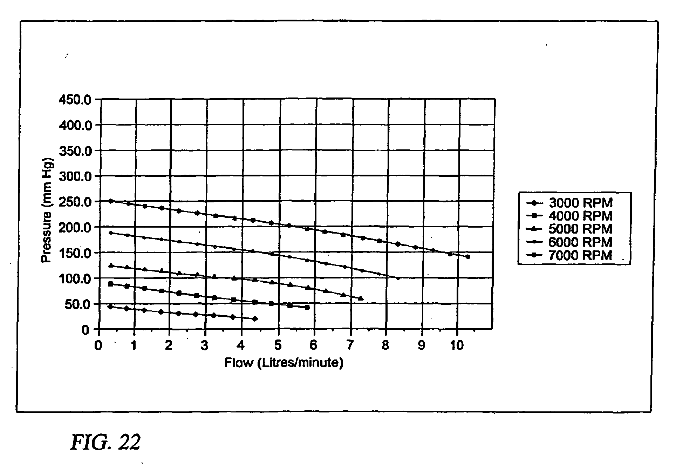

The dimensions and scale of the features and components comprising the centrifugal pump 26 of the present invention may be selected and/or adjusted over a wide range such that the centrifugal pump 26 may be dimensioned to be suitable for use in a wide variety of fluid pumping applications, including but not limited to pumping blood. When provided for use in pumping blood, the centrifugal pump 26 boasts a variety of advantageous characteristics, including but not limited to those shown and described by way of example with reference to FIG. 22. FIG. 22 is a graph illustrating the flow versus pressure characterization of the centrifugal pump 26 of the present invention dimensioned for use as a blood pump according to an exemplary of the present invention.

The control console 22 will now be described in detail with reference to FIGS. 1 and 23-24. The control console 22 is a microcomputer-based system for controlling the operation of the blood pump 26. The control console 22 is designed to operate on standard line voltage (AC) or an internal battery (DC) capable of supplying emergency backup power in case of AC power failure. The console 22 may be factory-wired for either 100-120V or 220-240V AC power input. The internal battery (not shown) is preferably a nickel cadmium rechargeable battery having a fully charged voltage of approximately 26 volts. As shown in FIG. 1, the control console 22 has a control line 40 coupled to the motor 20 to provide the necessary electrical signals to drive the motor 20. The motor 20 is preferably a brushless DC motor having a hollow cylindrical region (not shown) formed within a stator (not shown) for removably receiving the motor coupling assembly 100 of the magnetic cable drive assembly 30. With the magnetic cable drive assembly 30 coupled to the motor 20, the control console 22 may then be employed to create a rotating magnetic field to magnetically drive the magnet shaft assembly within the motor coupling assembly 100.

In a preferred embodiment, the speed of the motor 20 (and hence pump 26) may be adjusted based on feedback regarding the flow within the pump assembly 24. One manner of accomplishing this is through the use of the flow probe 48, which is preferably coupled to the inflow tubing 28 and communicatively linked to the control console 22 via control line 50 to aid in determining the flow rate produced by the centrifugal pump assembly 24. Flow probe 48 may comprise any number of commercially available flow determination devices, including but not limited to the flow probes available from Transonics, Inc. In a preferred embodiment, the flow probe 48 is capable of monitoring flow rates in the range between 0.5 liters/minute and 8.0 liters/minute. A display screen 302 is provided to display this flow rate information to an operator, along with other information (as will be discussed in greater detail below). Based on this flow feedback information, the flow rate may be adjusted by the operator through use of a speed adjustment knob 300 disposed on the front panel of the control console 22 for varying the speed of the motor 20 and thus the pump 26.

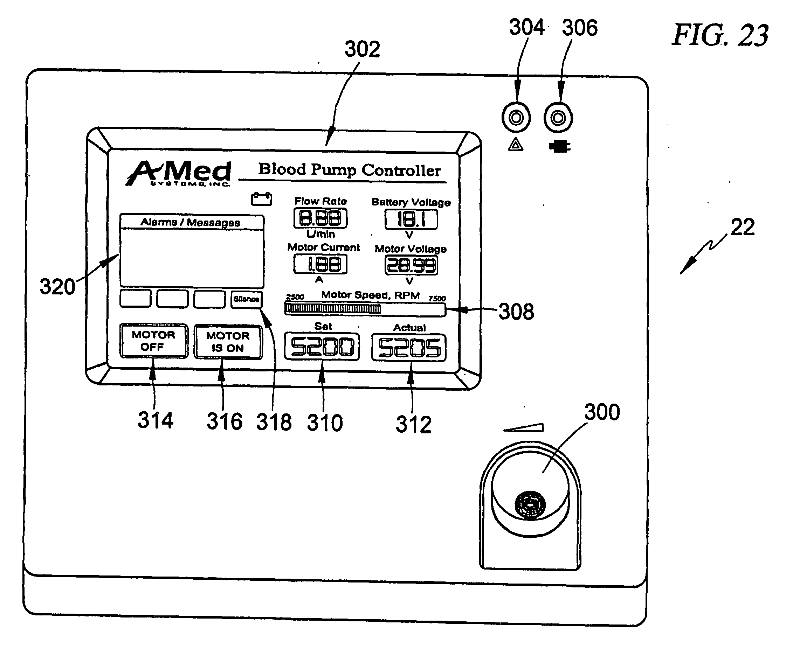

Referring to FIG. 23, the front panel of the control console 22 includes an alarm fault indicator 304 and power indicator 306 in addition to the speed adjustment knob 300 and screen display 302. The alarm fault indicator 304 is preferably a red light-emitting diode designed to turn on when any of a variety of alarm conditions (to be described below) occurs. The power indicator 306 is preferably a green light-emitting diode designed to turn on when the control console 22 is operating on AC power and to turn off when the control console 22 is operating on its internal battery. The display screen 302 includes a host of system status information to be visually communicated to an operator, including Flow Rate (liters/minute), Battery Voltage (volts), Motor Current (amperes), and Motor Voltage (volts). This system status information also includes a Motor Speed bar graph 308 designed to present a visual display (in green, preferably) of the motor speed within the preferred range of 2500 RPM to 7500 RPM. The Set display 310 illustrates the desired motor speed as set via the speed adjustment knob 300. The Actual display 312 illustrates the actual motor speed with a range of plus or minus 100 RPM.

The screen display 302 may also include a variety of controls (preferably touch-screen) for operating the control console 22. These include a Motor Off control 314 and a Motor On control 316. The Motor Off control 314 turns off the motor 20 and, when touched, preferably changes to light green and reads "MOTOR IS OFF." The Motor On control 316 turns on the motor 20 and, when touched, preferably changes to light green and reads "MOTOR IS ON." The control console 22 includes an audible alarm designed to turn on when any of a variety of alarm conditions occurs. A Silence control 318 is provided to temporarily turn off the audible alarm. When touched, the Silence control 318 will turn off the audible alarm and read "ALARM IS SILENCED." If the alarm condition persists for a predetermined period, such as 30 seconds, the audible alarm will turn on again and the Silence control 318 will read "SILENCE."

The display screen 302 includes an Alarm/Messages Display 320 for visually presenting various messages to the operator, including alarm indications and suggested actions. The alarm indications and suggested actions may include, but are not necessarily limited to, the following:

| | |
|---|---|
| Alarm Display | Suggested Actions |
| Low Flow Rate | Check Pump and Pump Cable |
| | Check Motor |
| | Check Flow Probe Cable |
| High Flow Rate | Check Pump |
| | Check Motor |
| Cannula Leakage | Check Cannula |
| Low Battery | Consider Replacing Battery |
| Motor Speed Mismatch | Check Connection to Pump |
| | Check Motor |
| High Motor Speed | Replace Motor |
| | Replace Controller |
| Low Motor Speed | Replace Motor |
| | Replace Controller |
| High Motor Current | Replace Motor |
| | Replace Controller |
| No Battery | Check Battery Connection |
| | Replace or Install Battery |
| High Controller Temp | Turn Off Controller |
| Touch-Screen Failure | Touch-Screen is Not Usable |

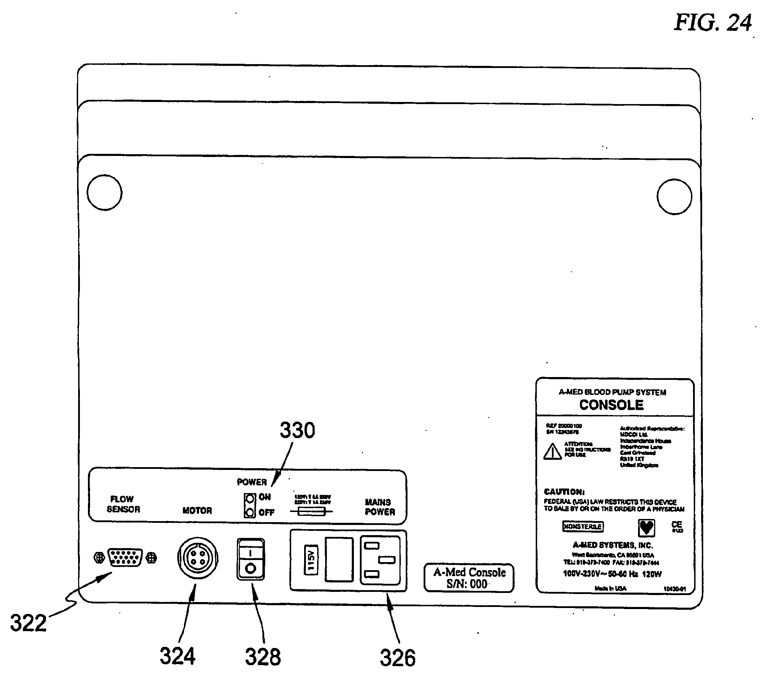

With reference to FIG. 24, the back of the control console 22 includes a plurality of connectors, including a flow probe connector 322, a motor connector 324, and an AC power connector 326. The flow probe connector 322 is designed to receive the control line 50 for communicatively coupling the flow probe 48 to the control console 22. The motor connector 324 is designed to receive the control line 40 for communicatively coupling the motor 20 to the control console 22. A power switch 328 is also provided for turning the control console 22 on and off. A dual-LED display 330 is provided in conjunction with the power switch 328 to visually indicate whether the power is on or off. Although not shown, the control console 22 includes an internal fan to cool the circuitry along with air vents formed preferably along the bottom and sides of the control console 22.

Use of the control console 22 in conjunction with the coaxial cannula assembly 12 of the present invention will not be discussed. To prepare the control console 22 for use, a power cord (such as 332 in FIG. 1) must be connected to the AC power connector 326 on the back of the control console 22. After checking to ensure the AC power is set to the proper voltage (via line voltage indicator on AC power connector 326), the power cord 332 may be plugged into the AC power source. The motor 20 should then be mounted near the patient, such as on the positioning assembly 38 shown in FIG. 1. The positioning assembly 38 is to be disposed near or within the surgical field. As described above, this allows the centrifugal pump assembly 24 to be positioned close to the patient so as to reduce the amount of tubing the blood is exposed to during the pumping process, thereby advantageously minimizing hemolysis. Once the motor 20 is positioned in this fashion, the control cable 40 can be employed to communicatively couple the control console 22 to the motor 20. This coupling process is facilitated by providing the control cable 40 with proximal and distal fittings that "click" into respective receptacles in the control console 22 and motor 20. The motor coupling assembly 100 of the magnetic cable drive assembly 30 may then be introduced into the hollow receptacle (not shown) within the motor 20 (as described above) to magnetically couple the impeller assembly 76 of the pump 26 to the motor 20. The flow probe 48 should then be coupled along the inlet tubing 28 of the pump assembly 24 and the control line 50 connected to the flow probe connector 322 on the back of the control console 22. The centrifugal blood pump assembly 24 may then be coupled to the coaxial cannula assembly 12 as described in detail above.

With the coaxial cannula assembly 12 disposed within the heart (as described above), the control console 22 may be turned on using the power switch 328. In a preferred embodiment, the control console 22 is preprogrammed such that the motor 20 will automatically to start at 2500 RPM when the Motor On control 316 is activated independent of the position of the motor speed adjustment knob 300. From this point, the speed of the motor 20 may be increased by rotating the speed adjustment knob 300 clockwise, resulting in a motor speed ranging from approximately 2500 RPM to 7500 RPM in a preferred embodiment. Operating the motor 20 within this range preferably produces flow rates for the pump assembly 24 ranging approximately from 0.3 liters/minute to 8.0 liters/minute. A device may be employed to assess the cardiac output of the heart during the surgical procedure such that an operator can adjust the flow rate of the pump assembly 24 (by controlling motor 20) to ensure the patient's cardiac output is maintained at sufficient levels throughout the entire procedure. Such devices may comprise any of a variety of cardiac output monitoring devices or systems, including but not limited to those employed in the esophagus, within the heart itself (such as in the aorta), and those affixed on the aorta. Exemplary cardiac output monitoring devices include those commercially available from Deltex Medical, Inc. and Transonics, Inc. Ensuring adequate cardiac output in this fashion is advantageous in that it prevents complications that may otherwise result, such as reduced perfusion of the vital organs during periods of lower cardiac output. Such reductions in cardiac output may result (in the absence of the present invention) due to kinking or collapse of the pulmonary artery, aorta, and/or portions of the ventricular and atrial walls during surgery. This can occur particularly when the heart is manipulated to perform coronary artery bypass graft (CABG) procedures on the coronary arteries on the posterior and/or lateral regions of the heart.

To disassemble the system following the surgical procedure, the inner cannula 16 should be clamped along the non-reinforced section 31 and the motor speed reduced to approximately 2500 RPM. The inlet tubing 28 should then be clamped. Clamping the coaxial cannula assembly 12 in this fashion prevents retrograde flow while the cannula assembly 12 is removed from the patient as described above. The motor 20 may now be turned off, such as by pressing the Motor Off control 314 on the screen display 302. The control console 22 may next be turned off via the power switch 328. The flow probe 48 may then be disconnected from the inlet tube 28 and the control console 22, after which point the pump assembly 24 may be disconnected from the motor 20 by removing the motor coupling assembly 100 of the magnetic cable drive assembly 30 from the hollow region of the motor 20. The motor 20 may then be disconnected from the control console 22 and removed from the positioning assembly 38, which can also be removed from its position near or within the surgical field.

It will be appreciated by those skilled in the art that changes could be made to the embodiments described above without departing from the broad inventive concepts thereof. It is understood, therefore, that this invention is not limited to the particular embodiments disclosed, but is intended to cover modifications within the scope of the present invention as defined by the appended claims.

## Claims

1. A cannulation assembly (10) for providing circulatory support daring surgical procedures, comprising:
a pumping system (14) including a centrifugal blood pump (26) having an inlet (32) and an outlet (34), a motor (20) coupled to a controller (22), and a cable drive assembly (30) extending between the motor (20) and the blood pump (26) for driving the blood pump (26) according to control signals communicated from the controller (22) to the motor (20); and
a cannula assembly (12) defining a first flow path for transporting blood between the pump (26) and a first predetermined location within the circulatory system of a patient, and a second flow path for transporting blood between the pump (26) and a second predetermined location within the circulatory system of a patient, the cannula assembly including an inner cannula (16) disposed within an outer cannula (18), the first flow path being defined between the exterior of the inner cannula (16) and the interior of the outer cannula (18), the second flow path being defined within the interior of the inner cannula (16), wherein the inner cannula (16) includes a wire-reinforced elongated section having an open distal end (46) and a non-reinforced clamping section (31), and wherein the outer cannula (18) includes a main tubular section (13), a fluid inlet section (44), and a curved distal section (56) haying an open end (62), and wherein inner cannula (16) is dimensioned to be slideably advanced through the outer cannula (18) such that the open distal end (46) of the inner cannula (16) extends a distance from the open distal end (62) of the curved distal section (56) of the outer cannula (18), wherein the cannula assembly (12) includes a stylet (11) having a rigid distal section removably insertable into the inner cannula (16), nuch that, in use, the stylet (11) can be preloaded within the whole length of the inner cannula (16).

2. The cannulation assembly (10) of claim 1, wherein the first and second flow paths of the cannula assembly (12) are slideably coupled to one another and dimensioned to extend, in use, into the respective first and second predetermined locations through a single incision formed in the vascular system of the patient.

3. The cannulation assembly (10) of claim 2, wherein an inlet and outlet of the first and second flow paths of the cannula assembly are disposed in a generally coaxial arrangement with the second flow path disposed at least partially within the first flow path.

4. The cannulation assembly (10) of claim 1, wherein the cannula assembly (12) and centrifugal blood pump (26) are equipped with quick-connect fittings for coupling and decoupling the first flow path to the inlet of the centrifugal blood pump (26) and the second flow path to the outlet of the centrifugal blood pump (26).

5. The cannulation assembly (10) of claim 1, wherein the centrifugal blood pump (26) has a priming port (36) for removing air from within the centrigual blood pump (26) in preparation for use.

6. The cannulation assembly (10) of claim 5, wherein the priming port (36) of the centrifugal blood pump (26) is dimensioned to receive a syringe capable of withdrawing air from within the centrifugal blood pump (26).

7. The cannulation assembly (10) of claim. 1, wherein the cable drive assembly (30) is dimensioned such that the centrifugal blood pump (26) may be disposed at or within a sterile surgical field.

8. The cannulation assembly (10) of claim 1, wherein the cable drive assembly (30) includes a magnetic coupling (100) dimensioned to be removably inserted into a lumen formed within a stator of the motor (20).

9. The cannulation assembly (10) of claim 1, wherein the motor (20) is coupled to the controller (22) via an electrical cable (40) dimensioned such that the motor (20) may be disposed at or within a sterile surgical field.

10. The cannulation assembly (10) of claim, 1, wherein the controller (22) includes a microcomputer programmed to regulate the speed of the motor (20).

11. The cannulation assembly (10) of claim 10, wherein the controller (22) controls the speed of the motor (20) based on feedback from a flow rate monitoring device coupled (48) to the centrifugal blood pump (26).

12. The cannulation assembly (10) of claim 11, wherein the flow rate monitoring device (48) is coupled to the inlet (32) of the centrifugal blood pump (26).

13. The cannulation assembly (10) of claim 10, wherein the controller (22) includes a manual speed adjustment control (300) such that an operator may cause the microcomputer to adjust the speed of the motor (20) to a range of approximately 2500 RPM to 7500 RPM.

14. The cannulation assembly (10) of claim 1, wherein the outer cannula (18) is dimensioned to be introduced into the heart such that the fluid inlet section (44) is disposed within the right atrium (54) and the curved distal section (56) is disposed in the right ventricle (60).

15. The cannulation assembly (10) of claim 14, wherein the curved distal section (56) of the outer cannula (18) is dimensioned to point in the general direction of the pulmonic valve (64) such that the inner cannula (16) may be slideably guided into the pulmonary artery (66) after the outer cannula (18) has been positioned within the heart.

## Patentansprüche

1. Kanülisier-Baugruppe (10) zum Bereitstellen einer zirkulierenden Unterstützung während chirurgischer Eingriffe mit
einem Pumpsystem (14), welches eine zentrifugale Blutpumpe (26) mit einem Einlass (32) und einem Auslass (34), einen Motor (20), der mit einer Steuer- oder Regeleinheit (22) gekoppelt ist, und eine Kabelantriebsbaugruppe (30), die sich zwischen dem Motor (20) und der Blutpumpe (26) zum Antrieb der Blutpumpe (26) entsprechend den Steuer- oder Regelsignalen, die von der Steuer- oder Regeleinheit (22) zu dem Motor (20) übertragen werden, erstreckt, aufweist; und
einer Kanülen-Baugruppe (12), die einen ersten Flusspfad zum Transportieren von Blut zwischen der Pumpe (26) und einem ersten vorbestimmten Ort in dem zirkulierenden System eines Patienten sowie einen zweiten Flusspfad zum Transportieren von Blut zwischen der Pumpe (26) und einem zweiten vorbestimmten Ort in dem zirkulierenden System eines Patienten definiert, wobei die Kanülen-Baugruppe eine innere Kanüle (16) aufweist, die in einer äußeren Kanüle (18) angeordnet ist, der erste Flusspfad vorgegeben ist zwischen dem Äußeren der inneren Kanüle (16) und dem Inneren der äußeren Kanüle (18), der zweite Flusspfad in dem Inneren der inneren Kanüle (16) vorgegeben ist, die innere Kanüle (16) einen drahtverstärkten länglichen Abschnitt besitzt mit einem offenen distalen Ende (46) und einem nicht verstärkten Klemmabschnitt (31), die äußere Kanüle (18) einen rohrförmigen Hauptabschnitt (13), einen Einlassabschnitt (44) für Fluid und einen gekrümmten oder kurvenförmigen distalen Abschnitt (56) mit einem offenen Ende (62) aufweist und die innere Kanüle (16) geeignet dimensioniert ist zur Ermöglichung einer gleitenden Bewegung durch die äußere Kanüle (18) derart, dass sich das offene distale Ende (46) der inneren Kanüle (16) einen Abstand aus dem offenen distalen Ende (62) des gekrümmten distalen Abschnittes (56) der äußeren Kanüle (18) erstreckt, wobei die Kanülen-Baugruppe (12) ein Stylet (11) besitzt mit einem festen oder steifen distalen Abschnitt, der oder das lösbar derart in die innere Kanüle (16) einsetzbar ist, dass das Stylet (11) im Gebrauch in der gesamten Länge der inneren Kanüle (16) vorgerastet, vorgeladen oder vorgespannt werden kann.

2. Kanülisier-Baugruppe (10) nach Anspruch 1, **dadurch gekennzeichnet, dass** der erste Flusspfad und der zweite Flusspfad der Kanülen-Baugruppe (12) unter Ermöglichung einer Gleitbewegung miteinander gekoppelt sind und derart dimensioniert sind, dass sich diese im Gebrauch durch eine einzige Inzision in das vaskuläre System des Patienten in den ersten bzw. zweiten vorbestimmten Ort erstrecken

3. Kanülisier-Baugruppe (10) nach Anspruch 2, **dadurch gekennzeichnet, dass** ein Einlass und ein Auslass des ersten Flusspfades und des zweiten Flusspfades der Kanülen-Baugruppe grundsätzlich koaxialer zueinander angeordnet sind, wobei der zweite Flusspfad zumindest teilweise innerhalb des ersten Flusspfades angeordnet ist.

4. Kanülisier-Baugruppe (10) nach Anspruch 1, **dadurch gekennzeichnet, dass** die Kanülen-Baugruppe (12) und die zentrifugale Blutpumpe (26) mit Schnellverbindungs-Anschusselementen ausgestattet sind, um ein Koppeln und Entkoppeln des ersten Flusspfades mit dem Einlass der zentrifugalen Blutpumpe (26) und des zweiten Flusspfades mit dem Auslass der zentrifugalen Blutpumpe (26) zu ermöglichen.

5. Kanülisier-Baugruppe (10) gemäß Anspruch 1, **dadurch gekennzeichnet, dass** die zentrifugale Blutpumpe (26) einen vorbereitenden Anschluss (36) besitzt, der einer Entfernung von Luft aus dem Inneren der zentrifugalen Blutpumpe (26) zur Vorbereitung eines Einsatzes dient.

6. Kanülisier-Baugruppe (10) nach Anspruch 5, **dadurch gekennzeichnet, dass** der vorbereitende Anschluss (36) der zentrifugalen Blutpumpe (26) derart dimensioniert ist, dass dieser eine Spritze aufnehmen oder ein Spritze an diesen angeschlossen werden kann, die in der Lage ist, Luft aus dem Inneren der zentrifugalen Blutpumpe (26) zurückzuziehen oder zu entfernen.

7. Kanülisier-Baugruppe (10) nach Anspruch 1, **dadurch gekennzeichnet, dass** die Kabelantriebsbaugruppe (30) derart dimensioniert ist, dass die zentrifugale Blutpumpe (26) bei oder in einem sterilen chirurgischen Einsatzgebiet angeordnet werden kann.

8. Kanülisier-Baugruppe (10) nach Anspruch 1, **dadurch gekennzeichnet, dass** die Kabelantriebsbaugruppe (30) eine magnetische Kopplung (100) aufweist, die derart dimensioniert ist, dass diese lösbar in ein Lumen einsetzbar ist, welches in einem Stator des Motors (20) gebildet ist.

9. Kanülisier-Baugruppe (10) nach Anspruch 1, **dadurch gekennzeichnet, dass** der Motor (20) mit der Steuer- oder Regeleinheit (22) über ein elektrisches Kabel (40) gekoppelt ist, welches derart dimensioniert ist, dass der Motor (20) bei oder in einem sterilen chirurgischen Einsatzgebiet angeordnet werden kann.

10. Kanülisier-Baugruppe (10) nach Anspruch 1, **dadurch gekennzeichnet, dass** die Steuer- oder Regeleinheit (22) einen Mikrocomputer aufweist, der geeignet zur Regulierung der Geschwindigkeit des Motors (20) programmiert ist.

11. Kanülisier-Baugruppe (10) nach Anspruch 10, **dadurch gekennzeichnet, dass** die Steuer- oder Regeleinheit (22) die Geschwindigkeit des Motors (20) steuert oder regelt auf Basis einer Rückführung einer Überwachungseinrichtung für die Flussrate, die mit der zentrifugalen Blutpumpe (26) gekoppelt ist.

12. Kanülisier-Baugruppe (10) nach Anspruch 11, **dadurch gekennzeichnet, dass** die Überwachungseinrichtung (48) für die Flussrate mit dem Einlass (32) der zentrifugalen Blutpumpe (26) gekoppelt ist.

13. Kanülisier-Baugruppe (10) nach Anspruch 10, **dadurch gekennzeichnet, dass** die Steuer- oder Regeleinheit (22) eine manuelle Anpasseinheit oder Anpassregelung (300) für die Geschwindigkeit besitzt, so dass ein Betreiber verursachen kann, dass der Mikrocomputer die Geschwindigkeit des Motors (20) auf einen Bereich von ungefähr 2500 Umdrehungen pro Minute bis 7500 U/min anpasst.

14. Kanülisier-Baugruppe (10) nach Anspruch 1, **dadurch gekennzeichnet, dass** die äußere Kanüle (18) geeignet dimensioniert ist, so dass eine Einführung in das Herz derart möglich ist, dass der Einlassabschnitt (44) für Fluid in dem rechten Atrium (54) angeordnet ist und der gekrümmte distale Abschnitt (56) in dem rechten Ventrikel (60) angeordnet ist.

15. Kanülisier-Baugruppe (10) nach Anspruch 14, **dadurch gekennzeichnet, dass** der gekrümmte distale Abschnitt (56) der äußeren Kanüle (18) geeignet dimensioniert ist, um derart in die grundsätzliche Richtung der pulmonaren Klappe (64) zu weisen, dass die innere Kanüle (16) gleitend in die pulmonare Arterie (66) geführt werden kann, nachdem die äußere Kanüle (18) in dem Herz positioniert worden ist.

## Revendications

1. Ensemble de canulation (10) afin de fournir une assistance circulatoire pendant des interventions chirurgicales, comprenant :
un système de pompage (14) incluant une pompe sanguine centrifuge (26) ayant un orifice d'entrée (32) et un orifice de sortie (34), un moteur (20) couplé à un contrôleur (22), et un ensemble d'entraînement par câble (30) s'étendant entre le moteur (20) et la pompe sanguine (26) pour actionner la pompe sanguine (26) en fonction des signaux de commande communiqués par le contrôleur (22) au moteur (20) ; et
un ensemble formant une canule (12) définissant une première voie de passage pour transporter le sang entre la pompe (26) et un premier emplacement prédéterminé à l'intérieur du système circulatoire d'un patient, et une deuxième voie de passage pour transporter le sang entre la pompe (26) et un deuxième emplacement prédéterminé à l'intérieur du système circulatoire d'un patient, l'ensemble formant une canule comprenant une canule interne (16) disposée à l'intérieur d'une canule externe (18), la première voie de passage étant définie entre l'extérieur de la canule interne (16) et l'intérieur de la canule externe (18), la deuxième voie de passage étant définie à l'intérieur de la canule interne (16), dans lequel la canule interne (16) comprend une section allongée renforcée par une armature métallique ayant une extrémité distale ouverte (46) et une section de clampage non renforcée (31), et dans lequel la canule externe (18) comprend une section tubulaire principale (13), une section d'entrée des fluides (44), et une section distale recourbée (56) ayant une extrémité ouverte (62), et
dans lequel la canule interne (16) est dimensionnée de manière à pouvoir avancer par coulissage à travers la canule externe (18) de telle sorte que l'extrémité distale ouverte (46) de la canule interne (16) s'étende d'une distance allant de l'extrémité distale ouverte (62) de la section distale recourbée (56) de la canule externe (18), dans lequel l'ensemble formant une canule (12) inclut un stylet (11) ayant une section distale rigide pouvant être insérée de manière amovible dans la canule interne (16), de telle sorte que, en utilisation, le stylet (11) peut être préchargé à l'intérieur de toute la longueur la canule interne (16).

2. Ensemble de canulation (10) selon la revendication 1, dans lequel les première et deuxième voies de passage de l'ensemble formant une canule (12) sont couplées entre elles de manière coulissable et dimensionnées pour s'étendre, en utilisation, dans les premier et deuxième emplacements prédéterminés respectifs par le biais d'une simple incision ménagée dans le système vasculaire du patient.

3. Ensemble de canulation (10) selon la revendication 2, dans lequel un orifice d'entrée et un orifice de sortie de la première et de la deuxième voie de passage de l'ensemble formant une canule sont disposés selon une disposition généralement coaxial e, avec la deuxième voie de passage qui est disposée au moins partiellement à l'intérieur de la première voie de passage.

4. Ensemble de canulation (10) selon la revendication 1, dans lequel l'ensemble formant une canule (12) et la pompe sanguine centrifuge (26) sont équipés de raccords à connexion rapide pour coupler et découpler la première voie de passage à l'orifice d'entrée de la pompe sanguine centrifuge (26) et la deuxième voie de passage à l'orifice de sortie de la pompe sanguine centrifuge (26).

5. Ensemble de canulation (10) selon la revendication 1, dans lequel la pompe sanguine centrifuge (26) comporte un port d'amorçage (36) pour éliminer l'air présent à l'intérieur de la pompe sanguine centrifuge (26) au cours des préparatifs d'utilisation.

6. Ensemble de canulation (10) selon la revendication 5, dans lequel le port d'amorçage (36) de la pompe sanguine centrifuge (26) est dimensionné pour recevoir une seringue capable d'aspirer l'air présent à l'intérieur de la pompe sanguine centrifuge (26).

7. Ensemble de canulation (10) selon la revendication 1, dans lequel l'ensemble d'entraînement par câble (30) est dimensionné de telle sorte que la pompe sanguine centrifuge (26) peut être disposée au niveau ou à l'intérieur d'un champ chirurgical stérile.

8. Ensemble de canulation (10) selon la revendication 1, dans lequel l'ensemble d'entraînement par câble (30) comprend un couplage magnétique (100) dimensionné pour être inséré de manière amovible dans une lumière formée à l'intérieur d'un stator du moteur (20).

9. Ensemble de canulation (10) selon la revendication 1, dans lequel le moteur (20) est couplé au contrôleur (22) par le biais d'un câble électrique (40) dimensionné de telle sorte que le moteur (20) peut être disposé au niveau ou à l'intérieur d'un champ chirurgical stérile.

10. Ensemble de canulation (10) selon la revendication 1, dans lequel le contrôleur (22) comprend un micro-ordinateur programmé pour réguler la vitesse du moteur (20).

11. Ensemble de canulation (10) selon la revendication 10, dans lequel le contrôleur (22) contrôle la vitesse du moteur (20) à partir d'un retour d'information en provenance d'un dispositif de surveillance du débit (48) couplé à la pompe sanguine centrifuge (26).

12. Ensemble de canulation (10) selon la revendication 11, dans lequel le dispositif de surveillance du débit (48) est couplé à l'orifice d'entrée (32) de la pompe sanguine centrifuge (26).

13. Ensemble de canulation (10) selon la revendication 10, dans lequel le contrôleur (22) comprend une commande de réglage manuel de la vitesse (300) de telle sorte qu'un opérateur peut commander au micro-ordinateur d'ajuster la vitesse du moteur (20) dans une plage d'approximativement 2500 tours/minute à 7500 tours/minute.

14. Ensemble de canulation (10) selon la revendication 1, dans lequel la canule externe (18) est dimensionnée pour être introduite dans le coeur de telle sorte que la section d'entrée des fluides (44) est disposée à l'intérieur de l'oreillette droite (54) et la section distale recourbée (56) est disposée à l'intérieur du ventricule droit (60).

15. Ensemble de canulation (10) selon la revendication 14, dans lequel la section distale recourbée (56) de la canule externe (18) est dimensionnée pour pointer dans la direction générale de la valvule pulmonaire (64) de telle sorte que la canule interne (16) peut être guidée de manière coulissable dans l'artère pulmonaire (66) après positionnement de la canule externe (18) à l'intérieur du coeur.
